# EUROPEAN PATENT APPLICATION

(11) **EP 4 299 125 A1**
(43) Date of publication of application: **03.01.2024**
(21) Application number: 22305967.6
(22) Date of filing: 30.06.2022
(51) Int. Cl.: A61P 25/00, A61K 51/10, C07K 16/28, A61K 39/395

(54) **ANTI-MGLUR2 BIPARATOPIC NANOBODIES AND USES THEREOF**

(71) Applicant: UNIVERSITE DE MONTPELLIER, 34090 Montpellier (FR); Centre National de la Recherche Scientifique (CNRS), 75016 Paris (FR); Institut National de la Santé et de la Recherche Médicale (INSERM), 75013 Paris (FR); Institut Jean Paoli & Irène Calmettes, 13009 Marseille (FR); Université d'Aix Marseille, 13007 Marseille (FR)
(72) Inventor: CHAMES, Patrick, 13012 MARSEILLE (FR); KNIAZEFF, Julie, 34730 PRADES-LE-LEZ (FR); OOSTERLAKEN, Mathieu, 34090 MONTPELLIER (FR); ROGLIARDO, Angelina, 34090 MONTPELLIER (FR); BECAMEL, Carine, 34270 SAINT-MATHIEU-DE-TREVIERS (FR); PIN, Jean-Philippe, 34190 CAZILHAC (FR); RONDARD, Philippe, 34980 SAINT-GÉLY-DU-FESC (FR)
(74) Representative: Regimbeau

(57) **Abstract**

The present invention relates to a biparatopic nanobody comprising :
(i) one single domain antibody having
a CDR1 having a sequence set forth as SEQ ID NO:1, a CDR2 having a sequence set forth as SEQ ID NO:2and a CDR3 having a sequence set forth as SEQ ID NO:3; or a CDR1 having a sequence set forth as SEQ ID NO:4, a CDR2 having a sequence set forth as SEQ ID NO:5 and a CDR3 having a sequence set forth as SEQ ID NO:6; or a CDR1 having a sequence set forth as SEQ ID NO:7, a CDR2 having a sequence set forth as SEQ ID NO:8 and a CDR3 having a sequence set forth as SEQ ID NO:9; preferably
a CDR1 having a sequence set forth as SEQ ID NO:1, a CDR2 having a sequence set forth as SEQ ID NO:2 and a CDR3 having a sequence set forth as SEQ ID NO:3; and

(ii) another single domain antibody having
a CDR1 having a sequence set forth as SEQ ID NO:10, a CDR2 having a sequence set forth as SEQ ID NO:11 and a CDR3 having a sequence set forth as SEQ ID NO:12; or
a CDR1 having a sequence set forth as SEQ ID NO:13, a CDR2 having a sequence set forth as SEQ ID NO:14 and a CDR3 having a sequence set forth as SEQ ID NO:15; or a CDR1 having a sequence set forth as SEQ ID NO:16, a CDR2 having a sequence set forth as SEQ ID NO:17 and a CDR3 having a sequence set forth as SEQ ID NO:18.

## Description

### TECHNICAL FIELD OF THE INVENTION

The present invention relates to anti-mGluR2 biparatopic nanobodies and uses thereof.

### BACKGROUND ART

Glutamate is the major amino acid neurotransmitter in the mammalian central nervous system. Furthermore, glutamate is at the center of several different neurological and psychiatric diseases, where there is an imbalance in glutamatergic neurotransmission. Glutamate activates metabotropic glutamate receptors (mGluRs) which have a more modulatory role that contributes to the fine-tuning of synaptic efficacy. The mGluR2 subtype is negatively coupled to adenylate cyclase via activation of Gai-protein, and its activation leads to inhibition of glutamate release in the synapse.

Activating mGluR2 was shown in clinical trials to be efficacious to treat anxiety disorders. In addition, activating mGluR2 in various animal models was shown to be efficacious, thus representing a potential novel therapeutic approach for the treatment of schizophrenia, epilepsy, addiction/drug or alcohol dependence, Parkinson's disease, pain, sleep disorders and Huntington's disease.

Most brain diseases are still in need of new drugs. To date, mostly small molecules are used and developed for these pathologies, but they sometimes lack selectivity or show off-target binding resulting in side effects.

Immunotherapies have shown to be efficient in many medical areas, but there is no antibody-based therapeutics for a brain disease to date, mostly due to their little brain penetration. The nanobodies, that are the variable fragment of camelids heavy chain antibodies, have the ability to modulate protein activity but their efficacy in reducing the symptoms of a brain disorder after peripheral administration is still lacking.

So there is still a need to develop new antibody-based therapeutics for a brain disease.

The Applicant provides a new bivalent nanobody, also named biparatopic nanobody, that penetrates the brain and modulates the activity of the metabotropic glutamate receptor type 2 leading to positive behavioral effect in an animal model of schizophrenia. It improves the cognition for at least 7 days after a single intraperitoneal injection. These new results open avenues for nanobody-based therapeutic strategies for the treatment of brain disorders.

### SUMMARY OF THE INVENTION

The present invention relates to a biparatopic nanobody comprising
(i) one single domain antibody having
   a CDR1 having a sequence set forth as SEQ ID NO:1, a CDR2 having a sequence set forth as SEQ ID NO:2 and a CDR3 having a sequence set forth as SEQ ID NO:3; or a CDR1 having a sequence set forth as SEQ ID NO:4, a CDR2 having a sequence set forth as SEQ ID NO:5 and a CDR3 having a sequence set forth as SEQ ID NO:6; or a CDR1 having a sequence set forth as SEQ ID NO:7, a CDR2 having a sequence set forth as SEQ ID NO:8 and a CDR3 having a sequence set forth as SEQ ID NO9; preferably
   a CDR1 having a sequence set forth as SEQ ID NO:1, a CDR2 having a sequence set forth as SEQ ID NO:2 and a CDR3 having a sequence set forth as SEQ ID NO:3; and
(ii) another single domain antibody having
   a CDR1 having a sequence set forth as SEQ ID NO:10, a CDR2 having a sequence set forth as SEQ ID NO:11 and a CDR3 having a sequence set forth as SEQ ID NO:12; or
   a CDR1 having a sequence set forth as SEQ ID NO:13, a CDR2 having a sequence set forth as SEQ ID NO:14 and a CDR3 having a sequence set forth as SEQ ID NO:15; or a CDR1 having a sequence set forth as SEQ ID NO:16, a CDR2 having a sequence set forth as SEQ ID NO:17 and a CDR3 having a sequence set forth as SEQ ID NO:18.

Another subject matter is a nucleic acid encoding for a biparatopic nanobody according to the invention, preferably a nucleic acid having at least 80% of identity with the sequence set forth as SEQ ID NO:40 or 41.

The invention also relates to a vector which comprises the above nucleic acid.

Another subject-matter is a host cell which is transformed with the nucleic acid sequence or the vector disclosed above.

Another subject-matter is the biparatopic nanobody according to the invention for use as a medicament, in particular for use in a method of treating a patient suffering from a neurological or psychiatric disorder associated with glutamate dysfunction.

The invention also relates to a pharmaceutical composition comprising a biparatopic nanobody according to the invention and a pharmaceutical excipient.

Another subject-matter of the invention is a biparatopic nanobody according to the invention which is conjugated with a detectable label.

The present invention also relates to an *in vitro* method of detecting the activation of mGluR2 in a sample comprising the steps of i) contacting the sample with a biparatopic nanobody conjugated with a detectable label as defined above, ii) and detecting the binding of said biparatopic nanoboby to said sample wherein said detection is indicative of the activation of mGluR2.

### DESCRIPTION OF THE FIGURES

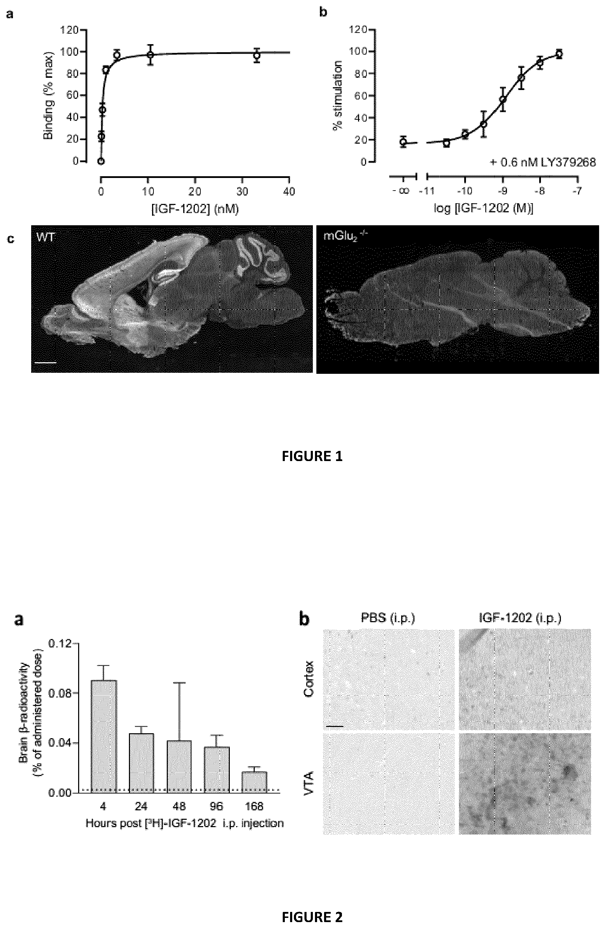

**Figure 1****. IGF1202 is a high affinity and specific bivalent biparatopic mGlu2 nanobody with PAM properties. a** Schematic representation of IGF1168 and IGF1202 in which the two nanobodies are linked using the hinge from llama HcAb (EPKIPQPQPKPQPQPQPQPQPKPQPKPEP= SEQ ID NO:25= linker L1). **b** Saturation binding curves of IGF1168 and IGF1202 on HEK293 cells co-expressing SNAP-mGlu2 and the high affinity glutamate transporter EAAC1, in the presence of the agonist LY379268 (1 µM). Data are mean ± SD of three individual experiments each performed in triplicates. **c** Potentiation of IP1 accumulation induced by EC₂₀ of LY379268 in cells co-expressing mGlu2, EAAC1 and GqTop. Data are mean ± SD of three individual experiments each performed in triplicates. **d** Ex-vivo immunofluorescence on brain coronal sections from wild-type (left) or mGlu2 ^{-/-} (right) mice using a red-labled IGF1202 (100 nM). The bar represents 1.1 mm.
**Figure 2****. IGF1202 biparatopic nanobody penetrates the brain. a** Quantification of the radioactivity present in the brain 4h, 24h, 48h, 4 or 7 days after i.p. administration of [³H]-IGF1202 (3 µCi) in mice. Data are mean ± SD from three independant experiments. **b** Immunohistochemical detection of IGF1202 in the VTA (top) and in the cortex (bottom) 24 h after i.p. administration of IGF1202 (right) versus PBS (left). *VTA: ventral tegmental area.* The bar represents 50 µm.

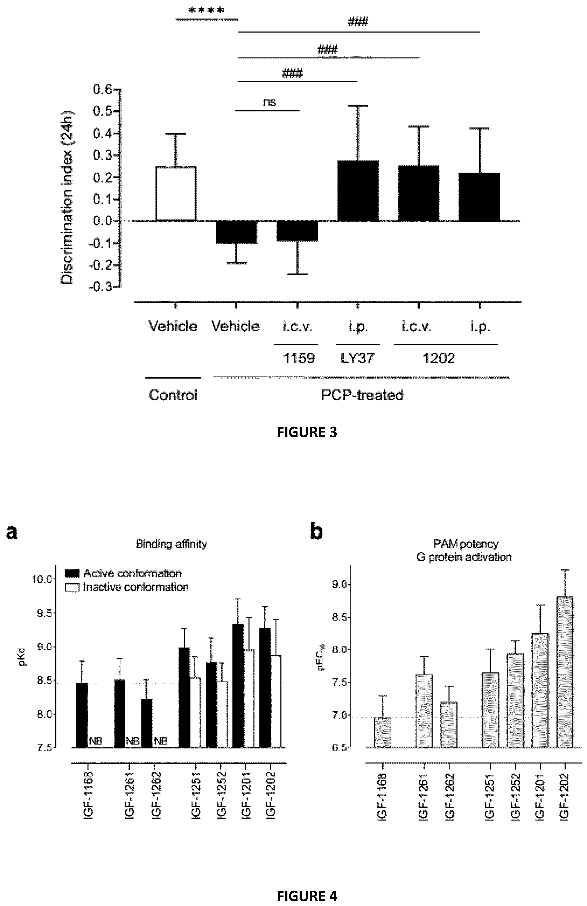

**Figure 3****. IGF1202 restores the score in novel object recognition (NOR) task in a neurodevelopmental PCP-induced mouse model**. Discrimination index of the time spent with a novel and a familiar object in the NOR test. Mice were treated at P7, P9 and P11 either saline (white) or PCP (10 mg/kg) (black). The NOR test was performed on adult animals. IGF1202 administration i.c.v (4 pmol, 5 µL) or i.p. (10 mg/kg) is as efficient as the reference mGlu2 agonist (LY379268, 1 mg/kg i.p.) to increase the discrimination index. IGF1159 (10 mg/kg i.c.v.) was used as negative control. Bars represent means ± SD; n= 9 - 27 per group. Statistics are calculated using one-way ANOVA, ####: comparison of PCP-treated versus control mice with p < 0.0001; ***: comparison the indicated compounds versus in PCP-treated mice with p < 0.001; ns: not statistically different.
**Figure 4****. Bivalent mGlu2 nanobodies and their pKd and pEC50 values compared to IGF1168. a-** Binding affinity on active (left) or inactive (center) conformation of the mGlu2 receptor and PAM potency (right) of all the bivalent nanobodies. Binding affinity for mGlu2 receptor was determined in a FRET-based assay (Scholler et al, 2017) where the active and inactive conformations of the receptor were stabilized by 1 µM of LY379268 or 1 µM of LY349268, respectively. The reference has been set the Kd value of IGF1168 binding for the active conformation and of IGF1159 binding (not shown, Scholler et al) for the inactive conformation. **b-** The PAM potency was evaluated by measuring the accumulation of IP1 using the IPOne kit in the presence of a saturation concentration of agonist (0.6 nM of LY379268 (EC₂₀)) in cells expressing the receptor, the glutamate transporter EAAC1 and the chimeric GqTop. Each datapoint represents the meam ± s.d. of an individual experiment performed in triplicate.

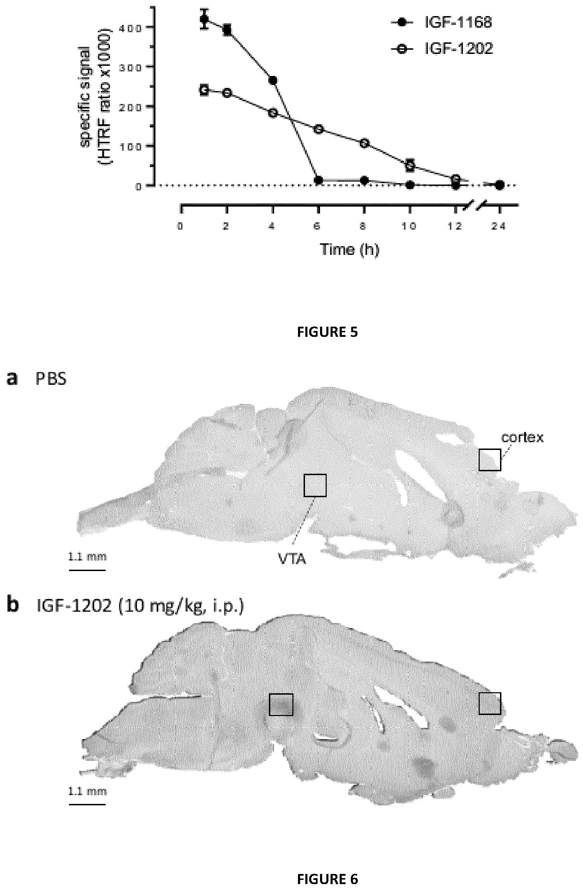

**Figure 5****. IGF1168 and IGF1202 residence time in the blood stream**. Time course determination of IGF1202 and IGF1168 in the blood stream after i.p administration of 10 mg/kg. HTRF values of diluted plasma samples were converted to nanobody concentration using the linear regression from panel b. Each data point is the mean ± s.d. of 3 experimental points.
**Figure 6****. Brain targeting of IGF1202 visualized using immunohistrochemistry**. Representative sagittal slices of immunohistochemical labeling using an anti-6His antibody from mice intraperitoneally administrated with PBS (a) or 10 mg/kg of IGF1202 (b) and sacrificed 24 h after administration. IGF1202 contains a C-terminal 6His tag. Boxes indicate areas that were magnified in Figure 2b.

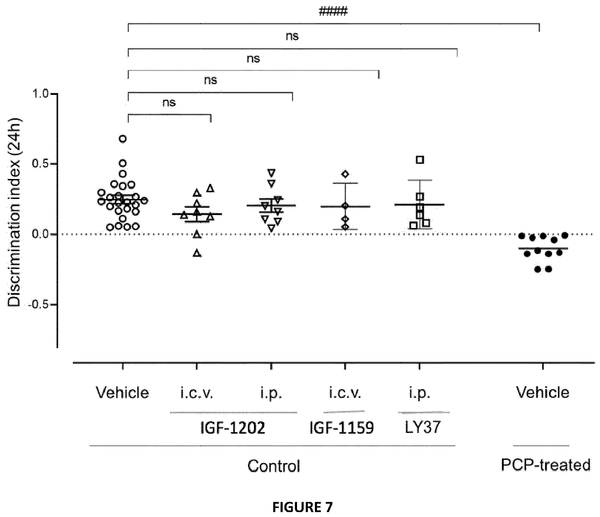

**Figure 7****. IGF1202 did not alter the score in novel object recognition (NOR) of control animals**. Discrimination index of the time spent with a novel and a familiar object in the NOR test. Mice were treated at P7, P9 and P11 either saline or PCP (10 mg/kg). The NOR test was performed on adult animals. None of the administered compounds, IGF1202 i.c.v (4 pmol, 5 µL) or i.p. (10 mg/kg), mGlu2 agonist (LY379268, 1 mg/kg i.p.) or IGF1159 (10 mg/kg i.c.v.), modified the discrimination score compared to vehicle treated animals. Bars represent means ± SD; n=6 - 27 per group. Statistics are calculated using one-way ANOVA, ####: comparison of PCP-treated versus control mice with p < 0.0001; ns: no statistical difference compared to vehicle control mice.

### DETAILED DESCRIPTION OF THE INVENTION

The sequences illustrated below are listed in the following table:

**Table 1**

| **SEQ ID NO :** | **SEQUENCE** | **DESCRIPTION** |
|---|---|---|
| 1 | GRTFSSFA | CDR1 sequence of IGF-1159 nanobody |
| 2 | ISWSGST | CDR2 sequence of IGF-1159 nanobody |
| 3 | ALGKGSSYYYSPSGYDY | CDR3 sequence of IGF-1159 nanobody |
| 4 | GRTFSTYG | CDR1 sequence of IGF-1160 nanobody |
| 5 | INWRDDVT | CDR2 sequence of IGF-1160 nanobody |
| 6 | AAQGRGSFKSIFERSRYDS | CDR3 sequence of IGF-1160 nanobody |
| 7 | G RTFSDLA | CDR1 sequence of IGF-1164 nanobody |
| 8 | INWSGHSI | CDR2 sequence of IGF-1164 nanobody |
| 9 | AADTTGGTYKLRTKEYDY | CDR3 sequence of IGF-1164 nanobody |
| 10 | GRTFRPYG | CDR1 sequence of IGF-1163 nanobody |
| 11 | IIWSLGYT | CDR2 sequence of IGF-1163 nanobody |
| 12 | AARDRSSSEYDY | CDR3 sequence of IGF-1163 nanobody |
| 13 | GRTFSIYS | CDR1 sequence of IGF-1166 nanobody |
| 14 | ITWRREYT | CDR2 sequence of IGF-1166 nanobody |
| 15 | ALRPGLRDDLNY | CDR3 sequence of IGF-1166 nanobody |
| 16 | VRFFSINT | CDR1 sequence of IGF-1168 nanobody |
| 17 | ITSSGST | CDR2 sequence of IGF-1168 nanobody |
| 18 | HADYKYTTHNTA | CDR3 sequence of IGF-1168 nanobody |
| 19 | QVQLVQSGGGLVQAGGSLRLSCAASGRTFSSFAM AWFRQAPGKEREFVAAISWSGSTGHADSVKGRFT ITRDNAKNTVFLQMNSLKPEDTAVYYCALGKGSSY YYSPSGYDYWGQGTQVTVSS | amino acid sequence of IGF-1159 nanobody |
| 20 | EVQLVESGGGLVQAGDSLRLSCAASGRTFSTYGM AWFRQAPGKEREFVARINWRDDVTYYADSLKGRF TISRDKAKNTVYLQLDSLTPEDTAVYYCAAQGRGS FKSIFERSRYDSWGQGTQVTVSS | amino acid sequence of IGF-1160 nanobody |
| 21 | EVQLVESGGGLVQAGGSLRLSCAASGRTFSDLAM GWFRQAPGKEREFVAAINWSGHSIYYTDSVKARF TISRDNAKDTVYLQMNSLKSEDTAVYFCAADTTG GTYKLRTKEYDYRGQGTQVTVSS | amino acid sequence of IGF-1164 nanobody |
| 22 | QVQLVQSGGGYVQAGGSLSVSCAASGRTFRPYG VGWFRQAPGKEREFVAAIIWSLGYTIYADSVKGRF TISRDNAKNTVYLQMNSLKAEDTAVYYCAARDRSS SEYDYWGQGTQVTVSS | amino acid sequence of IGF-1163 nanobody |
| 23 | EVQLVESGGGVVQPGDSLRLSCVASGRTDSIYSMA WFRQAPGKEREFVAIITWRREYTNYEDSVRGRFTI SRDNAKNAVYLQMNKLKPEDTAVYYCALRPGLRD DLNYWGQGTQVTVSS | amino acid sequence of IGF-1166 nanobody |
| 24 | QVQLVQSGGGLVQAGGSLRLSCAASVRFFSINTMG WYRQAPGKQRELVADITSSGSTNYADSGKGRFTIS RDNAKNTVYLQMNRLKPEDTAVYYCHADYKYTTH NTAWGQGTQVTVSS | amino acid sequence of IGF-1168 nanobody |
| 25 | EPKIPQPQPKPQPQPQPQPQPKPQPKPEP | linker sequence L1 |
| 26 | GGGGS GGGGS GGGGS | linker sequence L2 |
| 27 | GGGGS GGGGS GGGGS GGGGS | linker sequence L3 |
| 28 | GGGGS | linker sequence L4 |
| 29 | GGS | linker sequence L5 |
| 30 | GGG | linker sequence L6 |
| 31 | GGGSS GGGSS GGGSS | linker sequence L7 |
| 32 | QVQLVQSGGGLVQAGGSLRLSCAASVRFFSINTMG WYRQAPGKQRELVADITSSGSTNYADSGKGRFTIS RDNAKNTVYLQMNRLKPEDTAVYYCHADYKYTTH NTAWGQGTQVTVSSEPKIPQPQPKPQPQPQPQPQ PKPQPKPEPQVQLVQSGGGLVQAGGSLRLSCAASG RTFSSFAMAWFRQAPGKEREFVAAISWSGSTGHAD SVKGRFTITRDNAKNTVFLQMNSLKPEDTAVYYCAL GKGSSYYYSPSGYDYWGQGTQVTVSS | Amino acid sequence of the biparatopic IGF1202 (construct IGF1168-linker sequenceL1-IGF1159) |
| 33 | QVQLVQSGGGLVQAGGSLRLSCAASVRFFSINTMG WYRQAPGKQRELVADITSSGSTNYADSGKGRFTIS RDNAKNTVYLQMNRLKPEDTAVYYCHADYKYTTH NTAWGQGTQVTVSSEPKIPQPQPKPQPQPQPQPQ PKPQPKPEPQVQLVQSGGGLVQAGGSLRLSCAASG | Amino acid sequence of the biparatopic IGF1202-tag6xHIS |
| | RTFSSFAMAWFRQAPGKEREFVAAISWSGSTGHAD SVKGRFTITRDNAKNTVFLQMNSLKPEDTAVYYCAL GKGSSYYYSPSGYDYWGQGTQVTVSSLEHHHHHH | |
| 34 | caggtgcagctggtgcagtctgggggaggattggtgcaggct gggggctctctgagactctcctgtgcagcctctggacgcacc tttagtagcttcgccatggcctggttccgccaggctccaggg aaggagcgtgagtttgtagcagctattagctggagtggtagt acaggccatgcagactctgtgaagggccgattcaccatcac cagagacaacgccaagaacacggtgtttctgcaaatgaaca gcctgaaacctgaggacacggccgtttattactgtgcactag gaaaggggagtagttactactacagtcctagtgggtatgact actggggccaggggacccaggtcaccgtctcctca | nucleic acid sequence of IGF-1159 nanobody |
| 35 | gaggtgcagctggtggagtctggtggaggattggtgcaggct ggggactctctgagactctcctgtgcagcctctggacgcacc tttagtacctatggcatggcctggttccgccaggctccgggg aaggagcgtgagtttgtggcgcgaattaactggcgcgatgat gtcacatactatgcagactccctgaagggccgattcaccatc tcccgagacaaagccaagaacacggtgtatctgcaactgga cagcctgactcctgaggacacggccgtttattactgtgcagc ccaaggtcgcggtagttttaaatcaatcttcgagcggtcgcg ctatgactcctggggccaggggacccaggtcaccgtctcct ca | nucleic acid sequence of IGF-1160 nanobody |
| 36 | gaggtgcagctggtggagtctgggggaggattggtgcaggct gggggctctctgagactgtcctgtgcagcctctggacgcacc ttcagtgaccttgccatgggctggttccgccaggctccagga aaggagcgtgagtttgtagcagccattaactggagtggtcat agcatatactatacagactccgtgaaggcccgattcaccatc tccagagacaacgccaaagacacggtgtatctgcaaatgaa cagcctgaaatctgaggacacggccgtttatttttgtgcagc agatacgactggtggcacctataaactccggacgaaggagt atgactaccgggggcaggggacccaggtcaccgtctcctca | nucleic acid sequence of IGF-1164 nanobody |
| 37 | caggtgcagctggtgcagtctgggggaggatatgtgcaggct gggggctctctgagcgtctcctgtgcagcctctggacgaacc ttccgtccctatggcgtgggctggttccgccaggctccaggg aaggagcgtgagtttgtagcagctattatttggagtcttggat acacaatttatgcagactccgtgaagggccgattcaccatct ccagagacaacgccaagaacacggtttatctgcaaatgaac agcctgaaagctgaggacacggccgtttattactgtgcagcc cgcgatcgtagttcgtcagagtat gactactggggccaggggacccaggtcaccgtctcctca | nucleic acid sequence of IGF-1163 nanobody |
| 38 | gaggtgcagctggtggagtctgggggaggagtggtgcagcct ggggattctctgagactctcttgtgtagcctctggacgcaccg atagtatttattccatggcctggttccgccaggctccagggaa ggaacgtgagtttgtagcgattattacctggagacgtgagtac acaaattatgaggactccgtgaggggccgattcaccatctcc agagacaacgccaagaacgcggtgtatctgcaaatgaacaa gttgaaacctgaggacacggccgtttattactgtgcacttagg ccaggcctacgcgatgacttaaactactggggccaggggacc caggtcaccgtctcctca | nucleic acid sequence of IGF-1166 nanobody |
| 39 | caggtgcagctggtgcagtctgggggaggcttggtgcaggct ggggggtctctgagactctcctgtgcagcctctgtaaggttct tcagtattaataccatgggctggtaccgccaggctccaggga agcagcgcgagttggtcgcagatattactagtagtggtagca | nucleic acid sequence of IGF-1168 nanobody |
| | caaactatgcagactccgggaagggccgattcaccatctcc agagacaacgccaagaacacggtgtatctgcagatgaaca ggttgaaacctgaggacacagccgtctattactgccatgcgg attacaaatacacaacccataacacggcctggggccagggg acccaggtcaccgtctcctca | |
| 40 | caggtgcagcttgtacagtctgggggaggcttggtgcaggct ggggggtctctgagactctcctgtgcagcctctgtaaggttct tcagtattaataccatgggctggtatcgccaggctccaggga agcagcgcgagttggtcgcagatattactagtagtggtagca caaactatgcagactccgggaagggccgattcaccatctcc agagacaacgccaagaacacggtgtatctgcagatgaaca ggttgaaacctgaggacacagccgtctattactgccatgcg gattacaaatacacaacccataacacggcctggggccagg gtacccaggtcaccgtctcctcagaaccgaagatcccgcag ccgcagccaaaacctcagccgcagccacagccgcaacctc aaccgaaaccgcaaccaaaaccggaacctcaggtgcaatt ggtgcagtctgggggaggattggtgcaggctgggggctctct gagactctcctgtgcagcctctggacgcacctttagtagctt cgccatggcctggttccgccaggctccagggaaggagcgtg agtttgtagcagctattagctggagtggtagtacaggccatg cagactctgtgaagggccgattcaccatcaccagagacaac gccaagaacacggtgtttctgcaaatgaacagcctgaaacc tgaggacacggccgtttattactgtgcactaggaaagggga gtagttactactacagtcctagtgggtatgactactggggcc aggggacccaggtcaccgtctcctca | nucleic acid sequence of the biparatopic IGF1202 (construct IGF1168-linker sequenceL1-IGF1159) |
| 41 | caggtgcagcttgtacagtctgggggaggcttggtgcaggct ggggggtctctgagactctcctgtgcagcctctgtaaggttct tcagtattaataccatgggctggtatcgccaggctccaggga agcagcgcgagttggtcgcagatattactagtagtggtagca caaactatgcagactccgggaagggccgattcaccatctcc agagacaacgccaagaacacggtgtatctgcagatgaaca ggttgaaacctgaggacacagccgtctattactgccatgcg gattacaaatacacaacccataacacggcctggggccagg gtacccaggtcaccgtctcctcagaaccgaagatcccgca gccgcagccaaaacctcagccgcagccacagccgcaacct caaccgaaaccgcaaccaaaaccggaacctcaggtgcaat tggtgcagtctgggggaggattggtgcaggctgggggctctc tgagactctcctgtgcagcctctggacgcacctttagtagctt cgccatggcctggttccgccaggctccagggaaggagcgtg agtttgtagcagctattagctggagtggtagtacaggccatg cagactctgtgaagggccgattcaccatcaccagagacaac gccaagaacacggtgtttctgcaaatgaacagcctgaaacc tgaggacacggccgtttattactgtgcactaggaaagggga gtagttactactacagtcctagtgggtatgactactggggcc aggggacccaggtcaccgtctcctcactcgagcaccaccac caccaccac | nucleic acid sequence of the biparatopic IGF1202-tag6xHIS |
| 42 | QVQLQESGGGLVQPGGSLRLSCEASGFTFSRFGMT WVRQAPGKGVEWVSGISSLGDSTLYADSVKGRFTI SRDNAKNTLYLQMNSLKPEDTAVYYCTIGGSLNPG GQGTQVTVSS | Amino acid sequence of the nanobody against Albumine (Alb8) |
| 43 | EVQLQASGGGLVQAGGSLRLSCSASVRTFSIYAM GWFRQAPGKEREFVAGINRSGDVTKYADFVKGR FSISRDNAKNMVYLQMNSLKPEDTALYYCAATWA | Amino acid sequence of the nanobody against Transferin receptor (Tfr) |
| | YDTVGALTSGYNFWGQGTQVTVSS | |
| 44 | QVQLVQSGGGLVQAGGSLRLSCAASVRFFSINTM GWYRQAPGKQRELVADITSSGSTNYADSGKGRF TISRDNAKNTVYLQMNRLKPEDTAVYYCHADYK YTTHNTAWGQGTQVTVSSGGGGSGGGGSGGG GSQVQLVQSGGGLVQAGGSLRLSCAASGRTFSS FAMAWFRQAPGKEREFVAAISWSGSTGHADSVK GRFTITRDNAKNTVFLQMNSLKPEDTAVYYCALG KGSSYYYSPSGYDYWGQGTQVTVSSGGGGSGG GGSEVQLQASGGGLVQAGGSLRLSCSASVRTFSI YAMGWFRQAPGKEREFVAGINRSGDVTKYADFV KGRFSISRDNAKNMVYLQMNSLKPEDTALYYCAA TWAYDTVGALTSGYNFWGQGTQVTVSS | Amino acid sequence of the biparatopic nanobody IGF-1267 (biparatopic IGF1201 with nanobody against Tfr in bold characters) |
| 45 | QVQLVQSGGGLVQAGGSLRLSCAASVRFFSINTM GWYRQAPGKQRELVADITSSGSTNYADSGKGRF TISRDNAKNTVYLQMNRLKPEDTAVYYCHADYKY TTHNTAWGQGTQVTVSSEPKIPQPQPKPQPQPQ PQPQPKPQPKPEPQVQLVQSGGGLVQAGGSLRL SCAASGRTFSSFAMAWFRQAPGKEREFVAAISWS GSTGHADSVKGRFTITRDNAKNTVFLQMNSLKPE DTAVYYCALGKGSSYYYSPSGYDYWGQGTQVTV SSGGGGSGGGGSEVQLQASGGGLVQAGGSLRLS CSASVRTFSIYAMGWFRQAPGKEREFVAGINRSG DVTKYADFVKGRFSISRDNAKNMVYLQMNSLKPE DTALYYCAATWAYDTVGALTSGYNFWGQGTQV TVSS | Amino acid sequence of the biparatopic nanobody IGF-1268 (biparatopic IGF1202 with nanobody against Tfr in bold characters) |
| 46 | QVQLVQSGGGLVQAGGSLRLSCAASVRFFSINTM GWYRQAPGKQRELVADITSSGSTNYADSGKGRF TISRDNAKNTVYLQMNRLKPEDTAVYYCHADYK YTTHNTAWGQGTQVTVSSGGGGSGGGGSGGG GSQVQLVQSGGGLVQAGGSLRLSCAASGRTFSS FAMAWFRQAPGKEREFVAAISWSGSTGHADSV KGRFTITRDNAKNTVFLQMNSLKPEDTAVYYCA LGKGSSYYYSPSGYDYWGQGTQVTVSSGGGGS GGGGSQVQLQESGGGLVQPGGSLRLSCEASGF TFSRFGMTWVRQAPGKGVEWVSGISSLGDSTLY ADSVKGRFTISRDNAKNTLYLQMNSLKPEDTAVY YCTIGGSLNPGGQGTQVTVSS | Amino acid sequence of the biparatopic nanobody IGF-1269 (biparatopic IGF1201 with nanobody against Alb8 in bold characters) |
| 47 | QVQLVQSGGGLVQAGGSLRLSCAASVRFFSINTM GWYRQAPGKQRELVADITSSGSTNYADSGKGRF TISRDNAKNTVYLQMNRLKPEDTAVYYCHADYK YTTHNTAWGQGTQVTVSSEPKIPQPQPKPQPQ PQPQPQPKPQPKPEPQVQLVQSGGGLVQAGG SLRLSCAASGRTFSSFAMAWFRQAPGKEREFVA AISWSGSTGHADSVKGRFTITRDNAKNTVFLQM NSLKPEDTAVYYCALGKGSSYYYSPSGYDYWGQ GTQVTVSSGGGGSGGGGSQVQLQESGGGLVQ PGGSLRLSCEASGFTFSRFGMTWVRQAPGKGV EWVSGISSLGDSTLYADSVKGRFTISRDNAKNTL YLQMNSLKPEDTAVYYCTIGGSLNPGGQGTQVT VSS | Amino acid sequence of the biparatopic nanobody IGF-1270 (biparatopic IGF1202 with nanobody against Alb8 in bold characters) |
| 48 | caggtgcagctgcaagaaagcggagggggcctggtccag ccaggaggcagcctccggctaagttgcgaggccagcggc | Nucleic acid sequence of the nanobody against Albumine (Alb8) |
| | ttcacattcagtagatttggcatgacatgggtcaggcagg ccccaggtaaaggcgtggaatgggtaagcggcataagca gtctgggagacagcacactctacgccgattccgtgaaag gccgcttcaccatctctagagacaacgcaaaaaataccc tgtaccttcagatgaattcactcaagcccgaagacacgg ccgtttattactgcacaatcggcgggtcactaaacccagg cggacaggggacacaagtaacggttagtagc | |
| 49 | gaagtgcagcttcaggctagcgggggaggcttggtgca ggctggggggtctctgagactctcctgtagcgcctctgtg cggacctttagcatctacgcaatgggctggttccgccagg ctccagggaaggaacgcgagttcgtcgcgggcatcaaca gaagtggtgacgtaacaaaatacgccgatttcgtaaagg ggcgattcagcatctccagagacaacgccaagaacatgg tgtatctgcagatgaacagcttgaaacctgaggacacag ccctgtattactgcgccgcgacctgggcgtacgacaccg tgggcgcgctgaccagcggctataatttttggggccaag gcacccaggtcaccgtgagctca | Nucleic acid sequence of the nanobody against Transferin receptor (Tfr) |
| 50 | caggtgcagcttgtacagtctgggggaggcttggtgcag gctggggggtctctgagactctcctgtgcagcctctgtaa ggttcttcagtattaataccatgggctggtatcgccagg ctccagggaagcagcgcgagttggtcgcagatattacta gtagtggtagcacaaactatgcagactccgggaagggc cgattcaccatctccagagacaacgccaagaacacggt gtatctgcagatgaacaggttgaaacctgaggacacag ccgtctattactgccatgcggattacaaatacacaaccc ataacacggcctggggccagggtacccaggtcaccgtc tcctcaggtgggggtggcagcggtggcggcggtagcggg ggcggtggcagccaggtgcaattggtgcagtctggggg aggattggtgcaggctgggggctctctgagactctcctg tgcagcctctggacgcacctttagtagcttcgccatggc ctggttccgccaggctccagggaaggagcgtgagtttgt agcagctattagctggagtggtagtacaggccatgcaga ctctgtgaagggccgattcaccatcaccagagacaacgc caagaacacggtgtttctgcaaatgaacagcctgaaacc tgaggacacggccgtttattactgtgcactaggaaagggg agtagttactactacagtcctagtgggtatgactactggg gccaggggacccaggtcaccgtctcctcaggtgggggtg gcagcggtggcggcggtagcgaagtgcagcttcaggct agcgggggaggcttggtgcaggctggggggtctctgaga ctctcctgtagcgcctctgtgcggacctttagcatctacgc aatgggctggttccgccaggctccagggaaggaacgcga gttcgtcgcgggcatcaacagaagtggtgacgtaacaaa atacgccgatttcgtaaaggggcgattcagcatctccaga gacaacgccaagaacatggtgtatctgcagatgaacagc ttgaaacctgaggacacagccctgtattactgcgccgcga cctgggcgtacgacaccgtgggcgcgctgaccagcggcta taatttttggggccaaggcacccaggtcaccgtgagctca | Nucleic acid sequence of the biparatopic nanobody IGF-1267 |
| 51 | caggtgcagcttgtacagtctgggggaggcttggtgcagg ctggggggtctctgagactctcctgtgcagcctctgtaagg ttcttcagtattaataccatgggctggtatcgccaggctcc agggaagcagcgcgagttggtcgcagatattactagtagt ggtagcacaaactatgcagactccgggaagggccgattc accatctccagagacaacgccaagaacacggtgtatctg | Nucleic acid sequence of the biparatopic nanobody IGF-1268 |
| | cagatgaacaggttgaaacctgaggacacagccgtctat tactgccatgcggattacaaatacacaacccataacacg gcctggggccagggtacccaggtcaccgtctcctcagaa ccgaagatcccgcagccgcagccaaaacctcagccgcag ccacagccgcaacctcaaccgaaaccgcaaccaaaaccg gaacctcaggtgcaattggtgcagtctgggggaggattgg tgcaggctgggggctctctgagactctcctgtgcagcctct ggacgcacctttagtagcttcgccatggcctggttccgcca ggctccagggaaggagcgtgagtttgtagcagctattagct ggagtggtagtacaggccatgcagactctgtgaagggccg attcaccatcaccagagacaacgccaagaacacggtgttt ctgcaaatgaacagcctgaaacctgaggacacggccgttt attactgtgcactaggaaaggggagtagttactactacagt cctagtgggtatgactactggggccaggggacccaggtca ccgtctcctcaggtgggggtggcagcggtggcggcggtag cgaagtgcagcttcaggctagcgggggaggcttggtgca ggctggggggtctctgagactctcctgtagcgcctctgtgc ggacctttagcatctacgcaatgggctggttccgccaggct ccagggaaggaacgcgagttcgtcgcgggcatcaacagaa gtggtgacgtaacaaaatacgccgatttcgtaaaggggcga ttcagcatctccagagacaacgccaagaacatggtgtatct gcagatgaacagcttgaaacctgaggacacagccctgtatt actgcgccgcgacctgggcgtacgacaccgtgggcgcgctg accagcggctataattttgggccaaggcacccaggtcac cgtgagctca | |
| 52 | caggtgcagcttgtacagtctgggggaggcttggtgcaggc tggggggtctctgagactctcctgtgcagcctctgtaaggtt cttcagtattaataccatgggctggtatcgccaggctccag ggaagcagcgcgagttggtcgcagatattactagtagtggt agcacaaactatgcagactccgggaagggccgattcacca tctccagagacaacgccaagaacacggtgtatctgcagat gaacaggttgaaacctgaggacacagccgtctattactgc catgcggattacaaatacacaacccataacacggcctggg gccagggtacccaggtcaccgtctcctcaggtgggggtgg cagcggtggcggcggtagcgggggcggtggcagccaggtg caattggtgcagtctgggggaggattggtgcaggctgggg gctctctgagactctcctgtgcagcctctggacgcaccttta gtagcttcgccatggcctggttccgccaggctccagggaag gagcgtgagtttgtagcagctattagctggagtggtagtac aggccatgcagactctgtgaagggccgattcaccatcacca gagacaacgccaagaacacggtgtttctgcaaatgaacagc ctgaaacctgaggacacggccgtttattactgtgcactagga aaggggagtagttactactacagtcctagtgggtatgactac tggggccaggggacccaggtcaccgtctcctcaggtggggg tggcagcggtggcggcggtagccaggtgcagctgcaagaaa gcggagggggcctggtccagccaggaggcagcctccggcta agttgcgaggccagcggcttcacattcagtagatttggcatg acatgggtcaggcaggccccaggtaaaggcgtggaatgggt aagcggcataagcagtctgggagacagcacactctacgccg attccgtgaaaggccgcttcaccatctctagagacaacgcaa aaaataccctgtaccttcagatgaattcactcaagcccgaag acacggccgtttattactgcacaatcggcgggtcactaaacc caggcggacaggggacacaagtaacggttagtagc | Nucleic acid sequence of the biparatopic nanobody IGF-1269 |
| 53 | caggtgcagcttgtacagtctgggggaggcttggtgcaggct ggggggtctctgagactctcctgtgcagcctctgtaaggttct tcagtattaataccatgggctggtatcgccaggctccaggga agcagcgcgagttggtcgcagatattactagtagtggtagca caaactatgcagactccgggaagggccgattcaccatctcc agagacaacgccaagaacacggtgtatctgcagatgaaca ggttgaaacctgaggacacagccgtctattactgccatgcg gattacaaatacacaacccataacacggcctggggccaggg tacccaggtcaccgtctcctcagaaccgaagatcccgcagc cgcagccaaaacctcagccgcagccacagccgcaacctcaa ccgaaaccgcaaccaaaaccggaacctcaggtgcaattggt gcagtctgggggaggattggtgcaggctgggggctctctgag actctcctgtgcagcctctggacgcacctttagtagcttcgcc atggcctggttccgccaggctccagggaaggagcgtgagttt gtagcagctattagctggagtggtagtacaggccatgcagac tctgtgaagggccgattcaccatcaccagagacaacgccaa gaacacggtgtttctgcaaatgaacagcctgaaacctgagga cacggccgtttattactgtgcactaggaaaggggagtagttac tactacagtcctagtgggtatgactactggggccaggggacc caggtcaccgtctcctcaggtgggggtggcagcggtggcggc ggtagccaggtgcagctgcaagaaagcggagggggcctggt ccagccaggaggcagcctccggctaagttgcgaggccagcg gcttcacattcagtagatttggcatgacatgggtcaggcaggc cccaggtaaaggcgtggaatgggtaagcggcataagcagtct gggagacagcacactctacgccgattccgtgaaaggccgctt caccatctctagagacaacgcaaaaaataccctgtaccttca gatgaattcactcaagcccgaagacacggccgtttattactg cacaatcggcgggtcactaaacccaggcggacaggggacac aagtaacggttagtagc | Nucleic acid sequence of the biparatopic nanobody IGF-1270 |

In particular, one single domain antibody ("IGF-1159 derivative") used in the present invention comprises a CDR1 having the sequence set forth as SEQ ID NO:1, a CDR2 having the sequence set forth as SEQ ID NO:2 and a CDR3 having the sequence set forth as SEQ ID NO:3.

In particular, one single domain antibody ("IGF-1160 derivative") used in the present invention comprises a CDR1 having the sequence set forth as SEQ ID NO:4, a CDR2 having the sequence set forth as SEQ ID NO:5 and a CDR3 having the sequence set forth as SEQ ID NO:6.

In particular, one single domain antibody (" IGF-1164 derivative") used in the present invention comprises a CDR1 having the sequence set forth as SEQ ID NO:7, a CDR2 having the sequence set forth as SEQ ID NO:8 and a CDR3 having the sequence set forth as SEQ ID NO:9.

In particular, one single domain antibody ("IGF-1163 derivative") used in the present invention comprises a CDR1 having the sequence set forth as SEQ ID NO:10, a CDR2 having the sequence set forth as SEQ ID NO:11 and a CDR3 having the sequence set forth as SEQ ID NO:12.

In particular, one single domain antibody ("IGF-1166 derivative") used in the present invention comprises a CDR1 having the sequence set forth as SEQ ID NO:13, a CDR2 having the sequence set forth as SEQ ID NO:14 and a CDR3 having the sequence set forth as SEQ ID NO:15.

In particular, one single domain antibody ("IGF-1168 derivative") used in the present invention comprises a CDR1 having the sequence set forth as SEQ ID NO:16, a CDR2 having the sequence set forth as SEQ ID NO:17 and a CDR3 having the sequence set forth as SEQ ID NO:18.

### Biparatopic nanobody

The present invention concerns a biparatopic nanobody comprising
(i) one single domain antibody having
   a CDR1 having a sequence set forth as SEQ ID NO:1, a CDR2 having a sequence set forth as SEQ ID NO:2 and a CDR3 having a sequence set forth as SEQ ID NO:3; or a CDR1 having a sequence set forth as SEQ ID NO:4, a CDR2 having a sequence set forth as SEQ ID NO:5 and a CDR3 having a sequence set forth as SEQ ID NO:6; or a CDR1 having a sequence set forth as SEQ ID NO:7, a CDR2 having a sequence set forth as SEQ ID NO:8 and a CDR3 having a sequence set forth as SEQ ID NO9; preferably
   a CDR1 having a sequence set forth as SEQ ID NO:1, a CDR2 having a sequence set forth as SEQ ID NO:2 and a CDR3 having a sequence set forth as SEQ ID NO:3; and
(ii) another single domain antibody having
   a CDR1 having a sequence set forth as SEQ ID NO:10, a CDR2 having a sequence set forth as SEQ ID NO:11 and a CDR3 having a sequence set forth as SEQ ID NO:12; or
   a CDR1 having a sequence set forth as SEQ ID NO:13, a CDR2 having a sequence set forth as SEQ ID NO:14 and a CDR3 having a sequence set forth as SEQ ID NO:15; or a CDR1 having a sequence set forth as SEQ ID NO:16, a CDR2 having a sequence set forth as SEQ ID NO:17 and a CDR3 having a sequence set forth as SEQ ID NO:18.

The term "bivalent" or "biparatopic" polypeptide or nanobody according to the invention means a nanobody comprising a single domain antibody and a second single domain antibody as herein defined, wherein these two single domain antibodies are capable of binding to two different epitopes of one antigen (e.g. mGluR2), which epitopes are not normally bound at the same time by one monospecific immunoglobulin, such as e.g. a conventional antibody or one single domain antibody.

In a particular embodiment, the biparatopic polypeptide of the invention comprises (i) one single domain antibody having a CDR1 having a sequence set forth as SEQ ID NO1, a CDR2 having a sequence set foth as SEQ ID NO:2 and a CDR3 having a sequence set forth as SEQ ID NO:3; and (ii) another single domain antibody having a CDR1 having a sequence set forth as SEQ ID NO:16, a CDR2 having a sequence set forth as SEQ ID NO:17 and a CDR3 having a sequence set forth as SEQ ID NO:18.

In another particular embodiment, the biparatopic polypeptide of the invention comprises (i) one single domain antibody having a CDR1 having a sequence set forth as SEQ ID NO:1, a CDR2 having a sequence set foth as SEQ ID NO:2 and a CDR3 having a sequence set forth as SEQ ID NO:3; and (ii) another single domain antibody having a CDR1 having a sequence set forth as SEQ ID NO:10, a CDR2 having a sequence set forth as SEQ ID NO:11 and a CDR3 having a sequence set forth as SEQ ID NO:12.

In another particular embodiment, the biparatopic polypeptide of the invention comprises (i) one single domain antibody having a CDR1 having a sequence set forth as SEQ ID NO:1, a CDR2 having a sequence set foth as SEQ ID NO:2and a CDR3 having a sequence set forth as SEQ ID NO:3; and (ii) another single domain antibody having a CDR1 having a sequence set forth as SEQ ID NO:13, a CDR2 having a sequence set forth as SEQ ID NO:14 and a CDR3 having a sequence set forth as SEQ ID NO:15.

In a particular embodiment, the biparatopic polypeptide of the invention comprises one single domain antibody having at least 85% identity, preferably at least 90% identity, more preferably at least 95% identity, and even 100% identity with the sequence set forth as SEQ ID NO:19 , and another single domain antibody having at least 85% identity, preferably at least 90% identity, more preferably at least 95% identity, and even 100% identity with the sequence set forth as SEQ ID NO:24.

In a particular embodiment, the biparatopic polypeptide of the invention comprises one single domain antibody having at least 85% identity, preferably at least 90% identity, more preferably at least 95% identity, and even 100% identity with the sequence set forth as SEQ ID NO:19 , and another single domain antibody having at least 85% identity, preferably at least 90% identity, more preferably at least 95% identity, and even 100% identity with the sequence set forth as SEQ ID NO:22.

In a particular embodiment, the biparatopic polypeptide of the invention comprises one single domain antibody having at least 85% identity, preferably at least 90% identity, more preferably at least 95% identity, and even 100% identity with the sequence set forth as SEQ ID NO:19 , and another single domain antibody having at least 85% identity, preferably at least 90% identity, more preferably at least 95% identity, and even 100% identity with the sequence set forth as SEQ ID NO:23.

According to the invention a first amino acid sequence having at least 85% of identity with a second amino acid sequence means that the first sequence has respectively 85; 86; 87; 88; 89; 90; 91; 92; 93; 94; 95; 96; 97; 98; or 99% of identity with the second amino acid sequence. Amino acid sequence identity is typically determined using a suitable sequence alignment algorithm and default parameters, such as BLAST P (Karlin and Altschul, 1990). The sequence identity is generally calculated by sequence alignment according to known methods in the art. To determine the percent identity of two amino acids sequences, the sequences are aligned for optimal comparison. For example, gaps can be introduced in the sequence of a first amino acid sequence for optimal alignment with the second amino acid sequence. The amino acid residues at corresponding amino acid positions are then compared. When a position in the first sequence is occupied by the same amino acid residue as the corresponding position in the second sequence, the molecules are identical at that position. The percent identity between the two sequences is a function of the number of identical positions shared by the sequences. Hence % identity = number of identical positions/total number of overlapping positions × 100.

In this comparison the sequences can be the same length or can be different in length. Optimal alignment of sequences for determining a comparison window may be conducted by the local homology algorithm of Smith and Waterman (J. Theor. Biol., 1981), by the homology alignment algorithm of Needleman and Wunsch (J. Mol. Biol, 1972)*,* by the search for similarity via the method of Pearson and Lipman (Proc. Natl. Acad. Sci. U.S.A., 1988), by computerized implementations of these algorithms (GAP, BESTFIT, FASTA and TFASTA in the Wisconsin Genetics Software Package Release 7.0, Genetic Computer Group, 575, Science Drive, Madison, Wisconsin) or for instance using publicly available computer software such as BLAST[2]. When using such software, the default parameters, e.g for gap penalty or extension penalty, are preferably used. The best alignment (i.e. resulting in the highest percentage of identity over the comparison window) generated by the various methods is selected.

### Linker

In some embodiments, the two single domain antibodies of the biparatopic nanobody of the present invention can be linked to each other directly (i.e. without use of a linker) or via a linker. The linker is typically a linker peptide and will, according to the invention, be selected so as to allow binding of the two single domain antibodies to each of their at at least two different epitopes of mGluR2. Suitable linkers inter alia depend on the epitopes and, specifically, the distance between the epitopes on mGluR2 to which the single domain antibodies bind, and will be clear to the skilled person based on the disclosure herein, optionally after some limited degree of routine experimentation. Suitable linkers are described herein in connection with specific nanobodies of the invention and may - for example and without limitation - comprise an amino acid sequence, which amino acid sequence preferably has a length of 9 or more amino acids, more preferably at least 17 amino acids, such as about 20 to 40 amino acids. However, the upper limit is not critical but is chosen for reasons of convenience regarding e.g. biopharmaceutical production of such nanobody. The linker sequence may be a naturally occurring sequence or a non-naturally occurring sequence. If used for therapeutical purposes, the linker is preferably non-immunogenic in the subject to which the anti-mGluR2 polypeptide of the invention is administered. One useful group of linker sequences are linkers derived from the hinge region of heavy chain antibodies as described in WO 96/34103 and WO 94/04678. Other examples are poly-alanine linker sequences such as Ala-Ala-Ala. Another preferred example of linker sequence is EPKIPQPQPKPQPQPQPQPQPKPQPKPEP (SEQ ID NO:25, also named linker L1 or 'HcAb'). Further preferred examples of linker sequences are Gly/Ser linkers of different length including (gly4ser)3 also named (GGGGS)₃, (gly4ser)4, (gly4ser), (gly3ser), gly3, and (gly3ser2)3.
SEQ ID NO:26 (GGGGS GGGGS GGGGS) also named Linker L2 or 'GS'
Linker L3: SEQ ID NO:27(GGGGS GGGGS GGGGS GGGGS)
Linker L4: SEQ ID NO: 28 (GGGGS)
Linker L5: SEQ ID NO:29 (GGS)
Linker L6: SEQ ID NO:30 (GGG)
Linker L7: SEQ ID NO:31 (GGGSS GGGSS GGGSS)

In a particular embodiment, the linker sequence has sequence selected from sequences set forth as SEQ ID NO:26 to SEQ ID NO: 31, preferably SEQ ID NO:26 (GGGGS GGGGS GGGGS).

In another a preferred embodiment, the first and second single domains are linked with a linker sequence having at least 80% of identity with the sequence set forth as SEQ ID NO:25, preferably 100% identity with SEQ ID NO: 25 (EPKIPQPQPKPQPQPQPQPQPKPQPKPEP).

The construction of the biparatopic nanobody may be either in a sense or a reverse sense, meaning that for each combination two single domain antibodies, we have 2 possible constructs.

The following table 2 lists the different contructs we may have wherein :
- the amino acid sequences of IGF-11xx nanobodies are defined as above ;
- the linkers are respectively 'HcAb' (EPKIPQPQPKPQPQPQPQPQPKPQPKPEP = SEQ ID NO:25) or 'GS' (GGGGS GGGGS GGGGS = SEQ ID NO: 26), and
- IGF-1159, IGF-1160 and IGF-1164 respectively are neutral nanobodies, mGlu2 specific; and IGF-1163, IGF-1166 and IGF-1168 respectively are PAM nanobodies, mGlu2 specific.

Advantageously, the biparatopic nanobody of the invention combined a neutral nanobody and a PAM nanobody.

In a particular and preferred embodiment, the neutral nanobody is IGF-1159.

In a particular and preferred embodiment, the PAM antibody is IGF-1168.

So in a particular and preferred embodiment, the biparatopic nanobody of the invention comprises IGF-1159 nanobody and IGF-1168, linked by a linker as defined above, and in particular the linker of SEQ ID NO:25, forming the biparatopic nanobody IGF-1202.

Bivalent nanobodies were obtained by fusing for example one copy of IGF1159 and IGF1168 to either the N- or C-terminus of IGF1168 using one of the two linkers. A 6His-tag was inserted at the C-terminus of the second nanobody for purification purposes. The sequences of the linkers are the following: hinge HcAb (EPKIPQPQPKPQPQPQPQPQPKPQPKPEP= SEQ ID NO: 25, also named 'HcAb'), (GGGGS)₃: GGGGSGGGGSGGGGS (= SEQ ID NO: 26, linker L2, also named 'GS')

**Table 2 illustrates a library of mGlu2 bivalent nanobodies generated with the non-limitative nanobodies and linkers illustrated in the invention**

| | |
|---|---|
| IGF-1159_{GS}**IGF-1159** | IGF-1191 |
| IGF-1159_{HcAb}**IGF-1159** | IGF-1192 |
| IGF-1160_{GS}**IGF-1159** | IGF-1193 |
| IGF-1160_{HcAb}**IGF-1159** | IGF-1194 |
| IGF-1163_{GS}**IGF-1159** | IGF-1195 |
| IGF-1163_{HcAb}**IGF-1159** | IGF-1196 |
| IGF-1164_{GS}**IGF-1159** | IGF-1197 |
| IGF-1164_{HcAb}**IGF-1159** | IGF-1198 |
| IGF-1166 _{GS}**IGF-1159** | IGF-1199 |
| IGF-1166 _{HcAb}**IGF-1159** | IGF-1200 |
| IGF-1168 _{GS}**IGF-1159** | IGF-1201 |
| IGF-1168 _{HcAb} **IGF-1159** | **IGF-1202** |
| | |
| IGF-1159_{GS}**IGF-1160** | IGF-1203 |
| IGF-1159_{HcAb}**IGF-1159** | IGF-1204 |
| IGF-1160_{GS}**IGF-1160** | IGF-1205 |
| IGF-1160_{HcAb}**IGF-1160** | IGF-1206 |
| IGF-1163_{GS}**IGF-1160** | IGF-1207 |
| IGF-1163_{HcAb}**IGF-1160** | IGF-1208 |
| IGF-1164_{GS}**IGF-1160** | IGF-1209 |
| IGF-1164_{HcAb}**IGF-1160** | IGF-1210 |
| IGF-1166_{GS}**IGF-1160** | IGF-1211 |
| IGF-1166_{HcAb}**IGF-1160** | IGF-1212 |
| IGF-1168_{GS}**IGF-1160** | IGF-1213 |
| IGF-1168_{HcAb}**IGF-1160** | IGF-1214 |
| | |
| IGF-1159_{GS}**IGF-1163** | IGF-1215 |
| IGF-1159_{HcAb}**IGF-1163** | IGF-1216 |
| IGF-1160_{GS}**IGF-1163** | IGF-1217 |
| IGF-1160_{HcAb}**IGF-1163** | IGF-1218 |
| IGF-1163_{GS}**IGF-1163** | IGF-1219 |
| IGF-1163_{HcAb}**IGF-1163** | IGF-1220 |
| IGF-1164_{GS}**IGF-1163** | IGF-1221 |
| IGF-1164_{HcAb}**IGF-1163** | IGF-1222 |
| IGF-1166_{GS}**IGF-1163** | IGF-1223 |
| IGF-1166_{HcAb}**IGF-1163** | IGF-1224 |
| IGF-1168_{GS}**IGF-1163** | IGF-1225 |
| IGF-1168_{HcAb}**IGF-1163** | IGF-1226 |
| | |
| IGF-1159_{GS}**IGF-1164** | IGF-1227 |
| IGF-1159_{HcAb}**IGF-1164** | IGF-1228 |
| IGF-1160_{GS}**IGF-1164** | IGF-1229 |
| IGF-1160_{HcAb}**IGF-1164** | IGF-1230 |
| IGF-1163_{GS}**IGF-1164** | IGF-1231 |
| IGF-1163_{HcAb}**IGF-1164** | IGF-1232 |
| IGF-1164_{GS}**IGF-1164** | IGF-1233 |
| IGF-1164_{HcAb}**IGF-1164** | IGF-1234 |
| IGF-1166_{GS}**IGF-1164** | IGF-1235 |
| IGF-1166_{HcAb}**IGF-1164** | IGF-1236 |
| IGF-1168_{GS}**IGF-1164** | IGF-1237 |
| IGF-1168_{HcAb}**IGF-1164** | IGF-1238 |
| | |
| IGF-1159_{GS}**IGF-1166** | IGF-1239 |
| IGF-1159_{HcAb}**IGF-1166** | IGF-1240 |
| IGF-1160_{GS}**IGF-1166** | IGF-1241 |
| IGF-1160_{HcAb}**IGF-1166** | IGF-1242 |
| IGF-1163_{GS}**IGF-1166** | IGF-1243 |
| IGF-1163_{HcAb}**IGF-1166** | IGF-1244 |
| IGF-1164_{GS}**IGF-1166** | IGF-1245 |
| IGF-1164_{HcAb}**IGF-1166** | IGF-1246 |
| IGF-1166_{GS}**IGF-1166** | IGF-1247 |
| IGF-1166_{HcAb}**IGF-1166** | IGF-1248 |
| IGF-1168_{GS}**IGF-1166** | IGF-1249 |
| IGF-1168_{HcAb}**IGF-1166** | IGF-1250 |
| | |
| IGF-1159_{GS}**IGF-1168** | IGF-1251 |
| IGF-1159_{HcAb}**IGF-1168** | IGF-1252 |
| IGF-1160_{GS}**IGF-1168** | IGF-1253 |
| IGF-1160_{HcAb}**IGF-1168** | IGF-1254 |
| IGF-1163_{GS}**IGF-1168** | IGF-1255 |
| IGF-1163_{HcAb}**IGF-1168** | IGF-1256 |
| IGF-1164_{GS}**IGF-1168** | IGF-1257 |
| IGF-1164_{HcAb}**IGF-1168** | IGF-1258 |
| IGF-1166_{GS}**IGF-1168** | IGF-1259 |
| IGF-1166_{HcAb}**IGF-1168** | IGF-1260 |
| IGF-1168_{GS}**IGF-1168** | IGF-1261 |
| IGF-1168_{HcAb}**IGF-1168** | IGF-1262 |
| **IGF-1201 -Tfr** | **IGF-1267** |
| **IGF-1202-Tfr** | **IGF-1268** |
| **IGF-1201-Alb8** | **IGF-1269** |
| **IGF-1202-Alb8** | **IGF-1270** |

In a particular embodiment, the following constructs are of particular interest:

| | |
|---|---|
| IGF-1168 _{GS}**IGF-1159** | IGF-1201 |
| IGF-1168 _{HcAb} **IGF-1159** | **IGF-1202** |
| **IGF-1201-Alb8** | **IGF-1269** |
| **IGF-1202-Alb8** | **IGF-1270** |

So in a particular embodiment, the biparatopic nanobody is selected in the group consisting of:
SEQ ID NO:19 (IGF-1159)- linker- SEQ ID NO: 24 (IGF-1168)
SEQ ID NO:24 (IGF1168)- linker- SEQ ID NO: 19 (IGF-1159)
SEQ ID NO:19 (IGF-1159)- linker - SEQ ID NO:22 (IGF-1163)
SEQ ID NO:22 (IGF-1163)- linker - SEQ ID NO:19 (IGF-1159)
SEQ ID NO:19 (IGF-1159)- linker - SEQ ID NO:23 (IGF-1166)
SEQ ID NO:23 (IGF-1166)- linker - SEQ ID NO:19 (IGF-1159)

In a particular and preferred embodiment, the biparatopic nanobody of the invention comprises the construct SEQ ID NO:24 (IGF-1168) - linker- SEQ ID NO: 19 (IGF-1159). The linker is preferably EPKIPQPQPKPQPQPQPQPQPKPQPKPEP (SEQ ID NO: 25).

In particular, the biparatopic nanobody of the invention comprises the amino acid sequence set forth as SEQ ID NO:32 also named IGF1202 corresponding to the construct IGF-1168 HcAb IGF-1159.

In another particular embodiment, the biparatopic nanobody of the invention comprises the amino acid sequence set forth as SEQ ID NO:33 (also named IGF1202-tag6xHIS).

In a particular and preferred embodiment, the biparatopic nanobody of the invention comprises an amino-acid sequence having at least 80% of identity with the sequence set forth as SEQ ID NO:32 or SEQ ID NO:33.

By at least 80% of identity, it means 80, 81, 82, 83, 84, 85, 86, 87, 88, 89, 90, 91, 92, 93, 94, 95, 96, 97, 98, 99, or 100% identity with the said sequences.

In the case of substitution of one or more consecutive or non-consecutive amino acids, substitutions are preferred in which the substituted amino acids are replaced by "equivalent" amino acids. Here, the expression "equivalent amino acids" is meant to indicate any amino acids likely to be substituted for one of the structural amino acids without however modifying the biological activities of the corresponding nanobodies or biparatopic nanobodies and of those specific examples defined below. Equivalent amino acids can be determined either on their structural homology with the amino acids for which they are substituted or on the results of comparative tests of biological activity between the various nanobodies likely to be generated.

As a non-limiting example, Table 3 below summarises the possible substitutions likely to be carried out without resulting in a significant modification of the biological activity of the corresponding modified antigen binding protein; inverse substitutions are naturally possible under the same conditions.

**Table 3**

| Original residue | Substitution(s) |
|---|---|
| Ala (A) | Val, Gly, Pro |
| Arg (R) | Lys, His |
| Asn (N) | Gln |
| Asp (D) | Glu |
| Cys (C) | Ser |
| Gln (Q) | Asn |
| Glu (E) | Asp |
| Gly (G) | Ala |
| His (H) | Arg |
| Ile (I) | Leu |
| Leu (L) | Ile, Val, Met |
| Lys (K) | Arg |
| Met (M) | Leu |
| Phe (F) | Tyr |
| Pro (P) | Ala |
| Ser (S) | Thr, Cys |
| Thr (T) | Ser |
| Trp (W) | Tyr |
| Tyr (Y) | Phe, Trp |
| Val (V) | Leu, Ala |

### Other binding domains

Also, the two single domain antibodies that bind to mGluR2 may also be linked to each other via a third single domain antibody (in which the two single domain antibodies may be linked directly to the third domain antibody or via suitable linkers). Such a third single domain antibody may for example be a single domain antibody that provides an increased half-life. For example, the latter single domain antibody may be a single domain antibody that is capable of binding to a (human) serum protein such as (human) serum albumin, as further described herein. In some embodiments, two or more single domain antibodies that bind to mGluR2 are linked in series (either directly or via a suitable linker) and the third (single) single domain antibody (which may provide for increased half-life, as described above) is connected directly or via a linker to one of these two or more aforementioned single domain antibodies.

In a particular embodiment, the biparatopic nanobody comprises a further domain, such as a peptide, a nanobody or a fluorinated chain, and in particular a further domain directed against a serum protein, preferably serum albumin.

In a particular embodiment, the biparatopic nanobody of the invention also comprises at least one further binding domain directed against another antigen (i.e. different from mGluR2). In some embodiments, the further binding domain is directed against a serum protein so that the half-life of the biparatopic nanobody is increased. Typically, said serum protein is albumin. In a particular embodiment, the further binding domain is directed against albumin, and may be a peptide (Stork et al., 2009), a nanobody (Walker et al., 2010; Hutt et al., 2012) or a fluorinated chain (Gerbier et al., 2017).

In a particular and preferred embodiment, the biparatopic nanobody of the invention is IGF1201-Alb8 or IGF1202-Alb8 comprising an amino acid sequence having at least 80% of identity with the sequence set forth SEQ ID NO:46 or SEQ ID NO:47, preferably IGF1202-Alb8 of SEQ ID NO:47.

As disclosed above, by at least 80% of identity, it means 80, 81, 82, 83, 84, 85, 86, 87, 88, 89, 90, 91, 92, 93, 94, 95, 96, 97, 98, 99, or 100% identity with the said sequence.

In some embodiments, the single domains antibodies are "humanized" single domain antibodies. As used herein the term "humanized" refers to a single domain antibody of the invention wherein an amino acid sequence that corresponds to the amino acid sequence of a naturally occurring VHH domain has been "humanized", i.e. by replacing one or more amino acid residues in the amino acid sequence of said naturally occurring VHH sequence (and in particular in the framework sequences) by one or more of the amino acid residues that occur at the corresponding position(s) in a VH domain from a conventional chain antibody from a human being. Methods for humanizing single domain antibodies are well known in the art. Typically, the humanizing substitutions should be chosen such that the resulting humanized single domain antibodies still retain the favourable properties of single domain antibodies of the invention. The one skilled in the art is able to determine and select suitable humanizing substitutions or suitable combinations of humanizing substitutions. For example, the single domain antibodies of the invention may be suitably humanized at any framework residue provided that the single domain antibodies remain soluble and do not significantly loss their affinity for mGluR2.

### Production of the nanobody

According to the invention, the nanobodies of the invention may be produced by conventional automated peptide synthesis methods or by recombinant expression. General principles for designing and making proteins are well known to those of skill in the art. In particular, the nanobodies are prepared as disclosed in EP 3 164 415 A2.

The nanobodies of the invention may be synthesized in solution or on a solid support in accordance with conventional techniques. Various automatic synthesizers are commercially available. The nanobodies of the invention may also be synthesized by solid-phase technology employing an exemplary peptide synthesizer such as a Model 433A from Applied Biosystems Inc. The purity of any given protein; generated through automated peptide synthesis or through recombinant methods may be determined using reverse phase HPLC analysis. Chemical authenticity of each peptide may be established by any method well known to those of skill in the art.

As an alternative to automated peptide synthesis, recombinant DNA technology may be employed wherein a nucleotide sequence which encodes a protein of choice is inserted into an expression vector, transformed or transfected into an appropriate host cell and cultivated under conditions suitable for expression as described herein below. Recombinant methods are especially preferred for producing longer polypeptides.

The present invention also relates to a nucleic acid sequence encoding for a biparatopic nanobody according to the invention, preferably a nucleic acid having at least 80% of identity with the sequence set forth as SEQ ID NO:40 (nucleic acid of biparatopic nanobody IGF1202) or SEQ ID NO:41 (nucleic acid of biparatopic nanobody IGF1202-tag6xHis). By 'at least 80% of identity', it means at least 80; 81; 82; 83; 84; 85; 86; 87; 88; 89; 90; 91; 92; 93; 94; 95; 96; 97; 98; 99; or 100% identity with the sequence set forth as SEQ ID NO:40 or 41, according to the definition disclosed above.

The invention also relates to a vector which comprises the said nucleic acid.

Another subject-matter of the invention is a host cell which is transformed with the said nucleic acid sequence or with the said vector.

A variety of expression vector/host systems may be utilized to contain and express the peptide or protein coding sequence. These include but are not limited to microorganisms such as bacteria transformed with recombinant bacteriophage, plasmid or cosmid DNA expression vectors; yeast transformed with yeast expression vectors (Giga-Hama et al., 1999); insect cell systems infected with virus expression vectors (e.g., baculovirus, see Ghosh et al., 2002); plant cell systems transfected with virus expression vectors (e.g., cauliflower mosaic virus, CaMV; tobacco mosaic virus, TMV) or transformed with bacterial expression vectors (e.g., Ti or pBR322 plasmid; see e.g., Babe et al., 2000); or animal cell systems. Those of skill in the art are aware of various techniques for optimizing mammalian expression of proteins, see e.g., Kaufman, 2000; Colosimo et al., 2000. Mammalian cells that are useful in recombinant protein productions include but are not limited to VERO cells, HeLa cells, Chinese hamster ovary (CHO) cell lines, COS cells (such as COS-7), W138, BHK, HepG2, 3T3, RIN, MDCK, A549, PC12, K562 and 293 cells. Exemplary protocols for the recombinant expression of the peptide substrates or fusion polypeptides in bacteria, yeast and other invertebrates are known to those of skill in the art and a briefly described herein below. Mammalian host systems for the expression of recombinant proteins also are well known to those of skill in the art. Host cell strains may be chosen for a particular ability to process the expressed protein or produce certain post-translation modifications that will be useful in providing protein activity. Such modifications of the polypeptide include, but are not limited to, acetylation, carboxylation, glycosylation, phosphorylation, lipidation and acylation. Post-translational processing which cleaves a "prepro" form of the protein may also be important for correct insertion, folding and/or function. Different host cells such as CHO, HeLa, MDCK, 293, WI38, and the like have specific cellular machinery and characteristic mechanisms for such post-translational activities and may be chosen to ensure the correct modification and processing of the introduced, foreign protein.

In the recombinant production of the nanobodies of the invention, it would be necessary to employ vectors comprising polynucleotide molecules for encoding the first and second single domains antibodies of the invention. Methods of preparing such vectors as well as producing host cells transformed with such vectors are well known to those skilled in the art. The polynucleotide molecules used in such an endeavor may be joined to a vector, which generally includes a selectable marker and an origin of replication, for propagation in a host. These elements of the expression constructs are well known to those of skill in the art. Generally, the expression vectors include DNA encoding the given protein being operably linked to suitable transcriptional or translational regulatory sequences, such as those derived from a mammalian, microbial, viral, or insect genes. Examples of regulatory sequences include transcriptional promoters, operators, or enhancers, mRNA ribosomal binding domains, and appropriate sequences which control transcription and translation.

The terms "expression vector," "expression construct" or "expression cassette" are used interchangeably throughout this specification and are meant to include any type of genetic construct containing a nucleic acid coding for a gene product in which part or all of the nucleic acid encoding sequence is capable of being transcribed.

The choice of a suitable expression vector for expression of the nanobodies of the invention will of course depend upon the specific host cell to be used, and is well known by those of skill in the art.

Expression requires that appropriate signals be provided in the vectors, such as enhancers/promoters from both viral and mammalian sources that may be used to drive expression of the nucleic acids of interest in host cells. Usually, the nucleic acid being expressed is under transcriptional control of a promoter. A "promoter" refers to a DNA sequence recognized by the synthetic machinery of the cell, or introduced synthetic machinery, required to initiate the specific transcription of a gene. Nucleotide sequences are operably linked when the regulatory sequence functionally relates to the DNA encoding the proteins of interest (e.g., both single domains antibodies). Thus, a promoter nucleotide sequence is operably linked to a given DNA sequence if the promoter nucleotide sequence directs the transcription of the sequence.

In a particular embodiment, for large scale nanobody production, pHEN plasmid vector with the bivalent construct of the invention are transformed in *E. coli* BL21DE3 strain (Thermofisher).

### Therapeutic uses and pharmaceutical composition

A further aspect of the present invention relates to a biparatopic nanobody as positive allosteric modulators of the metabotropic glutamate receptor subtype 2 ("mGluR2") and which are useful for the treatment or prevention of neurological and psychiatric disorders associated with glutamate dysfunction and diseases in which the mGluR2 subtype of metabotropic receptors is involved.

The biparatopic nanobodies of the present invention are positive allosteric modulators of metabotropic glutamate receptors, in particular they are positive allosteric modulators of mGluR2. The nanobodies of the present invention do not appear to bind to the glutamate recognition site, the orthosteric ligand site, but instead to an allosteric site within the extracellular domain of the receptor. In the presence of glutamate or an agonist of mGluR2, the nanobodies of the present invention increase the mGluR2 response. The nanobodies of the present invention are expected to have their effect at mGluR2 by virtue of their ability to increase the response of such receptors to glutamate or mGluR2 agonists, enhancing the response generated by the receptor.

Hence, the present invention also relates to a biparatopic nanobody of the present invention for use as a medicament.

### Pharmaceutical composition

So an other subject-matter of the invention is a pharmaceutical composition comprising a biparatopic nanobody according to the invention, and a pharmaceutical excipient.

According to the invention, the biparatopic nanobody of the invention is administered to the patient with a therapeutically effective amount.

By a "therapeutically effective amount" is meant a sufficient amount of the biparatopic nanobody of the invention to treat the disease (e.g. central nervous system disorder) at a reasonable benefit/risk ratio applicable to any medical treatment. It will be understood that the total daily usage of the compounds and compositions of the present invention will be decided by the attending physician within the scope of sound medical judgment. The specific therapeutically effective dose level for any particular patient will depend upon a variety of factors including the age, body weight, general health, sex and diet of the patient; the time of administration, route of administration, and rate of excretion of the specific compound employed; the duration of the treatment; drugs used in combination or coincidental with the specific polypeptide employed; and like factors well known in the medical arts. For example, it is well known within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Typically, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the patient to be treated. A medicament typically contains from about 0.01 mg to about 500 mg of the active ingredient, typically from 1 mg to about 100 mg of the active ingredient. A therapeutically effective amount of the drug is ordinarily supplied at a dosage level from 0.0002 mg/kg to about 20 mg/kg of body weight per day, especially from about 0.001 mg/kg to 7 mg/kg of body weight per day.

The biparatopic nanobody of the invention may be combined with pharmaceutically acceptable excipients, and optionally sustained-release matrices, such as biodegradable polymers, to form pharmaceutical compositions. "Pharmaceutically" or "pharmaceutically acceptable" refer to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type. In the pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms. Typically, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Solutions comprising compounds of the invention as free base or pharmacologically acceptable salts can be prepared in water suitably mixed with a surfactant, such as hydroxypropyl cellulose. Dispersions can also be prepared in glycerol, liquid polyethylene glycols, and mixtures thereof and in oils. Under ordinary conditions of storage and use, these preparations contain a preservative to prevent the growth of microorganisms. The nanobody (or nucleic acid encoding thereof) can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin. Sterile injectable solutions are prepared by incorporating the active polypeptides in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject.

The present invention extends to the treatment of neurological and psychiatric disorders associated with glutamate dysfunction by administering a therapeutically effective amount of a positive allosteric modulator of mGluR2, including biparatopic nanobody of the present invention, in combination with an mGluR2 agonist or another agent for the treatment of a neurological or psychiatric disorder.

The biparatopic nanobody of the present invention may be utilized in combination with one or more other drugs in the treatment, prevention, control, amelioration, or reduction of risk of diseases or conditions for which single domain antibodies or polypeptides of the present invention or the other drugs may have utility, where the combination of the drugs together are safer or more effective than either drug alone.

So another subject-matter of the invention relates to a pharmaceutical composition that comprises the biparatopic nanonbody of the invention and another mGluR2 agonist or another agent for the treatment of a neurological or psychiatric disorder.

### Therapeutic uses

The present invention also relates to a biparatopic nanobody of the present invention for use in the treatment or prevention, in particular treatment, of a disease or a condition in a mammal, including a human, the treatment of which is affected or facilitated by the neuromodulatory effect of allosteric modulators of mGluR2, in particular positive allosteric modulators thereof.

As used herein, the terms "treating" or "treatment" or "alleviation" refers to therapeutic treatment, wherein the object is to prevent or slow down (lessen) the targeted disease. A subject is successfully "treated" for a particular disease, if after receiving a therapeutic amount of the biparatopic nanobody according to the invention, the subject shows observable and/or measurable reduction in or absence of one or more signs and symptoms of said disease. In a particular embodiment the treatment is a prophylactic treatment. The term "prophylactic treatment" as used herein, refers to any medical or public health procedure whose purpose is to prevent a disease. As used herein, the terms "prevent", "prevention" and "preventing" refer to the reduction in the risk of acquiring or developing a given condition, or the reduction or inhibition of the recurrence or said condition in a subject who is not ill, but who has been or may be near a subject with the disease. It is also to be appreciated that the various modes of treatment or prevention of medical conditions as described are intended to mean "substantial," which includes total but also less than total treatment or prevention, and wherein some biologically or medically relevant result is achieved.

In a particular embodiment, the biparatopic nanobody of the invention or the pharmaceutical composition comprising it are used in a method of treating a patient suffering from a neurological or psychiatric disorder associated with glutamate dysfunction.

In some embodiments, the neurological and psychiatric disorders associated with glutamate dysfunction, include one or more of the following conditions or diseases: acute neurological and psychiatric disorders such as, for example, cerebral deficits subsequent to cardiac bypass surgery and grafting, stroke, cerebral ischemia, spinal cord trauma, head trauma, perinatal hypoxia, cardiac arrest, hypoglycemic neuronal damage, dementia (including AIDS-induced dementia), Alzheimer's disease, Huntington's Chorea, amyotrophic lateral sclerosis, ocular damage, retinopathy, cognitive disorders, idiopathic and drug-induced Parkinson's disease, muscular spasms and disorders associated with muscular spasticity including tremors, epilepsy, convulsions, migraine (including migraine headache), urinary incontinence, substance tolerance, substance withdrawal (including substances such as, for example, opiates, nicotine, tobacco products, alcohol, benzodiazepines, cocaine, sedatives, hypnotics, etc.), psychosis, schizophrenia, anxiety (including generalized anxiety disorder, panic disorder, and obsessive compulsive disorder), mood disorders (including depression, mania, bipolar disorders), trigeminal neuralgia, hearing loss, tinnitus, macular degeneration of the eye, emesis, brain edema, pain (including acute and chronic states, severe pain, intractable pain, neuropathic pain, and post-traumatic pain), tardive dyskinesia, sleep disorders (including narcolepsy), attention deficit/hyperactivity disorder, and conduct disorder, preferably schizophrenia, anxiety, mood disorders and epilepsy, and substance-related disorder or addiction/drug or alcohol dependence.

In a particular and preferred embodiment, the neurological disorder associated with glutamate dysfunction is selected from the group consisting of cerebral ischemia, head trauma, neurodegeneration, Alzheimer's disease, epilepsy, and pain, or the psychiatric disorder associated with glutamate dysfunction is selected from the group consisting of psychosis, schizophrenia, anxiety, depression, and substance-related disorder or addiction/drug or alcohol dependence.

In a particular and preferred embodiment, the disorder is schizophrenia.

In some embodiments, the condition or disease is a central nervous system disorder selected from the group of anxiety disorders, psychotic disorders, personality disorders, substance-related disorders, eating disorders, mood disorders, migraine, epilepsy or convulsive disorders, childhood disorders, cognitive disorders, neurodegeneration, neurotoxicity and ischemia.

In some embodiments, the central nervous system disorder is an anxiety disorder, selected from the group of agoraphobia, generalized anxiety disorder (GAD), obsessive-compulsive disorder (OCD), panic disorder, posttraumatic stress disorder (PTSD), social phobia and other phobias.

In some embodiments, the central nervous system disorder is a psychotic disorder selected from the group of schizophrenia, delusional disorder, schizoaffective disorder, schizophreniform disorder and substance-induced psychotic disorder.

In some embodiments, the central nervous system disorder is a personality disorder selected from the group of obsessive-compulsive personality disorder and schizoid, schizotypal disorder.

In some embodiments, the central nervous system disorder is a substance-related disorder or addiction/drug or alcohol dependence selected from the group of alcohol abuse, alcohol dependence, alcohol withdrawal, alcohol withdrawal delirium, alcohol-induced psychotic disorder, amphetamine dependence, amphetamine withdrawal, cocaine dependence, cocaine withdrawal, nicotine dependence, nicotine withdrawal, opioid dependence and opioid withdrawal.

In some embodiments, the central nervous system disorder is epilepsy or a convulsive disorder selected from the group of generalized nonconvulsive epilepsy, generalized convulsive epilepsy, petit mal status epilepticus, grand mal status epilepticus, partial epilepsy with or without impairment of consciousness, infantile spasms, epilepsy partialis continua, and other forms of epilepsy.

Of the disorders mentioned above, the treatment of anxiety, schizophrenia, migraine, depression, and epilepsy are of particular importance. At present, the fourth edition of the Diagnostic & Statistical Manual of Mental Disorders (DSM-IV) of the American Psychiatric Association provides a diagnostic tool for the identification of the disorders described herein. The person skilled in the art will recognize that alternative nomenclatures, nosologies, and classification systems for neurological and psychiatric disorders described herein exist, and that these evolve with medical and scientific progresses. Because such positive allosteric modulators of mGluR2, including single domain antibodies or polypeptides of the present invention, enhance the response of mGluR2 to glutamate, it is an advantage that the present methods utilize endogenous glutamate.

The invention also relates to a method of treating a patient suffering from a neurological or psychiatric disorder associated with glutamate dysfunction comprising administering the patient with a therapeutically effective amount of a biparatopic nanobody of the present invention.

In particular, the neurological or psychiatric disorder associated with glutamate dysfunction is defined above.

In a particular embodiment, the biparatopic nanobody is administered to the patient in combination with a mGluR2 agonist.

### Other uses

In a further aspect of the present invention, the biparatopic nanobody of the present invention are used to detect the activation of the mGluR2.

Accordingly, the present invention also relates to a biparatopic nanobody of the present invention which is conjugated with a detectable label to form an anti-mGluR2 immuno conjugate.

Suitable detectable labels include, for example, a radioisotope, a fluorescent label, a chemiluminescent label, an enzyme label, a bio luminescent label or colloidal gold. Methods of making and detecting such detectably-labeled immunoconjugates are well-known to those of ordinary skill in the art, and are described in more detail below. For instance, the detectable label can be a radioisotope that is detected by autoradiography. Isotopes that are particularly useful for the purpose of the present invention are ³ H, ¹²⁵ I, ¹³¹ I, ³⁵S and ¹⁴C. Anti-mGluR2 immunoconjugates can also be labeled with a fluorescent compound. The presence of a fluorescently-labeled biparatopic nanobody of the present invention is determined by exposing the immuno conjugate to light of the proper wavelength and detecting the resultant fluorescence. Fluorescent labeling compounds include fluorescein isothiocyanate, rhodamine, phycoerytherin, phycocyanin, allophycocyanin, o-phthaldehyde and fluorescamine and Alexa Fluor dyes. Alternatively, anti-mGluR2 immunoconjugates can be detectably labeled by coupling a biparatopic nanobody of the present invention to a chemiluminescent compound. The presence of the chemiluminescent-tagged immuno conjugate is determined by detecting the presence of luminescence that arises during the course of a chemical reaction. Examples of chemiluminescent labeling compounds include luminol, isoluminol, an aromatic acridinium ester, an imidazole, an acridinium salt and an oxalate ester. Similarly, a bio luminescent compound can be used to label anti-mGluR2 immunoconjugates of the present invention. Bio luminescence is a type of chemiluminescence found in biological systems in which a catalytic protein increases the efficiency of the chemiluminescent reaction. The presence of a bioluminescent protein is determined by detecting the presence of luminescence. Bio luminescent compounds that are useful for labeling include luciferin, luciferase and aequorin. Alternatively, anti-mGluR2 immunoconjugates can be detectably labeled by linking an anti-mGluR2 biparatopic nanobody of the present invention to an enzyme. When the anti-mGluR2 -enzyme conjugate is incubated in the presence of the appropriate substrate, the enzyme moiety reacts with the substrate to produce a chemical moiety which can be detected, for example, by spectrophotometric, fluorometric or visual means. Examples of enzymes that can be used to detectably label polyspecific immunoconjugates include β-galactosidase, glucose oxidase, peroxidase and alkaline phosphatase. Those of skill in the art will know of other suitable labels which can be employed in accordance with the present invention. The binding of marker moieties to anti-mGluR2 biparatopic nanobody of the present invention can be accomplished using standard techniques known to the art. Moreover, the convenience and versatility of immunochemical detection can be enhanced by using anti-mGluR2 biparatopic nanobody of the present invention that have been conjugated with avidin, streptavidin, and biotin.

For example, said detection is performed by a TR-FRET method as described in WO2010125314. This method is based on the use of two antibodies labelled with a fluorophore donor or acceptor compatible with TR-FRET measurement. It can be either the same single domain antibody specific of the active conformation, or two different antibodies, such as two single domain antibodies specific of the active conformation, or one antibody specific of the active conformation and the other one not specific of the active conformation.

Accordingly the present invention also provides an *in vitro* method of detecting the activation of mGluR2 in a sample comprising the steps of i) contacting the sample with a biparatopic nanobody of the present invention, ii) and detecting the binding of said biparatopic nanobody to said sample wherein said detection is indicative of the activation of mGluR2. Typically, the sample comprises cells that express naturally or artificially mGluR2. For example said cells are cells that are transformed with a nucleic acid molecule encoding for mGluR2 or cells having an endogenous expression of mGluR2 (e.g. neurons).

The present invention also relates to the biparatopic nanobody for use as a biomolecule transporter crossing the blood-brain barrier (BBB).

In fact, the Applicant demonstrates that the IGF-1202 is able to cross the BBB.

In a particular embodiment, the biomolecule is a detectable label.

In another embodiment, the biomolecule is a therapeutic agent for the treatment of a neurological or psychiatric disorder.

The term "biomolecule" in the present invention means a biological molecule, and encompassess a peptide, a polypeptide or a protein, including an antibody.

The fact that a biparatopic polypeptide comprising two single domain antibody targeting mGluR2 is able to cross the BBB is the demonstration that a single domain antibody targeting mGluR2 is able to cross the BBB with a small molecule such as another single domain antibody targeting mGluR2 or anyother biomolecule.

In particular, the biomolecule will have a molecular weight ranging from 15 to 60 kDa, in particular from 15 to 50 kDa, and particularly from 15 to 35 kDa.

In a particular embodiment, for use in biological and/or diagnostic analysis, the biomolecule linked to the single domain antibody directed against mGluR2 or a polypeptide comprising at least one single domain antibody directed against mGluR2 used in the present invention, is a detectable label.

The detectable label may be directly linked to the nanobody or polypeptide used in the present invention, or linked via a linker such as the ones disclosed above.

Suitable detectable labels include, for example, a radioisotope, a fluorescent label, a chemiluminescent label, an enzyme label, a bio luminescent label or colloidal gold. Examples of detectable labels are disclosed above.

In a particular embodiment, the biparatopic nanobody as defined above and linked to at least one detectable label, is used in biological analysis and/or diagnostic analysis.

By 'biological analysis' according to the invention, it means in particular the transfer into the brain of labelled molecules for PET scan analysis or other brain imaging techniques, allowing the detection of specific markers in clinical studies, that may then be identified as diagnostic markers.

By 'diagnostic analysis' according to the invention, it means in particular the transfer into the brain of labelled molecules for PET scan analysis or other brain imaging techniques allowing the detection of markers for the diagnostic of specific brain pathologies, such as Alzheimer (β-amyloid plaques), Parkinson (alpha-synuclein aggregates) or other early markers for these pathologies.

In another particular embodiment, for use in therapy, the biomolecule linked to the biparatopic nanobody directed against mGluR2 used in the present invention, is a therapeutic agent for the treatment of a neurological or psychiatric disorder.

The therapeutic agent may be directly linked to the nanobody used in the present invention, or linked via a linker such as the ones disclosed above.

In a particular embodiment, the therapeutic agent for the treatment of a neurological or psychiatric disorder is selected in the group consisting of peptides, nanobodies, and chemical compounds.

In particular, the nanobodies used as biomolecule linked are nanobodies distinct from nanobodies directed against mGluR2.

As examples of peptides that may be used for the treatment of a neurological or psychiatric disorder, mention may be made of natural neuropeptides.

As examples of nanobodies distinct from nanobodies directed against mGluR2 that may be used for the treatment of a neurological or psychiatric disorder, mention may be made of nanobodies targeting β-amyloid plaques.

As examples of chemical compounds that may be used for the treatment of a neurological or psychiatric disorder, mention may be made of chemical compounds with poor brain penetration or of positive allosteric modulators of mGluR2. Various compounds have been previsouly described as mGluR2 positive allosteric modulators.

The present invention will be now illustrated with the non-limitative examples.

### EXAMPLES

### Example 1: Materials and methods

**Ethics**. Care of the animals and surgical procedures were performed according to the Directive 2010/63/EU of the European Parliament, which had been approved by the Ministry of Agriculture, France. The project was approved by the French National Ethics Committee for Animal Experimentation (CEEA) under the number APAFIS#41112016021613253432 (for tritiated nanobodies). All experiments were performed in accordance with relevant named guidelines and regulations.

Animals C57BL/6J wild-type (WT) mice (21.5 ± 0.3 g) were purchased from Janvier Labs. Animals were housed under a 12 h light/dark cycle, at 22 ± 1°C and 55 ± 10% of relative humidity. All animals get access to water and food *ad libitum.*

**Reagents, cell lines, antibodies and plasmids.** HEK-293 cells (ATCC, CRL-1573) were cultivated in DMEM or DMEM-GlutaMAX (Life technologies, Villebon-sur-Yvette, France) complemented with 10% (v/v) fetal bovine serum (Sigma-Aldrich, Saint-Quentin-Falavier, France). Absence of mycoplasma was routinely checked using MycoAlert Mycoplama detection kit (LT07-318, Lonza, Amboise, France) according to the manufacturer's protocol. The pRK5 plasmids encoding wild-type rat mGlu2 receptor subunit, with an HA-tag and a SNAP inserted just after the signal peptide, was previously described (Doumazane et al., 2011). All drugs (LY379268 and LY341495) were from Tocris (Bio-techne, Lille, France). All HTRF reagents, anti-6His-d2 antibodies (61HISDLA), SNAP-Lumi4-Tb, d2-NHS were kindly gifted by Cisbio (Codolet, France). Monoclonal rabbit-anti-6His and DAB peroxidase substrate kit and ABC peroxidase kit, (anti-rabbit-HRP) were purchased from Invitrogen and Vector Laboratories (Burlingame, US), respectively.

**Plasmid of nanobodies constructs**. The bivalent monospecific constructs with the two different linkers were synthesized by Genecust (Boynes, France) and cloned between Ndel and Xhol in a modified pHEN1 vector in which the sequence coding for the G3P protein has been removed. The bivalent biparatopic constructs were then generated by PCR and subcloned via silent restriction sites inserted in the framework regions FR1 and FR4.

**Production and purification of nanobodies**. For large scale nanobody production, plasmids encoding bivalent nanobodies were transformed in E. coli BL21DE3 strain (Life technologies). A single colony was grown into 10 mL of LB, supplemented with 100 µg.mL⁻¹ ampicillin, 1% (w/v) glucose, overnight at 37°C with shaking. Then 1 L of LB supplemented with around 10 mL of the preculture was incubated until an OD600 of 0.6 - 0.7. The nanobody expression was induced with 1 mM Isopropyl β-D-1-thiogalactopyranoside (IPTG) and bacteria were grown overnight at 28°C with shaking. Bacteria were then collected by centrifugation for 10 min at 5000 g, resuspended in 10 mL per 1 L culture of ice-cold TES buffer (0.2 M Tris, 0.5 mM EDTA, 0.5 M sucrose, pH 8) and incubated for at least 1 h at 4°C on shaking platform. Twenty milliliters per little of culture of TES/4 (TES buffer dilute 4 times in water) were the added to the solution and further incubated for at least 45 min at 4°C on a shaking platform. The periplasmic extract was recovered by collecting the supernatant after centrifugation of the suspension for 30 min at 10000 g at 4°C. The His-tagged nanobodies were then purified from the periplasmic extract by using Ni-NTA purification (Qiagen, Hilden, Germany) according to the manufacturer's instructions. Desalting was performed using disposable PD-10 desalting Columns (Cytiva, US) according to the manufacturer's instructions to obtain the nanobodies in phosphate buffer solution. Endotoxins were removed using Proteus NoEndoTM S (Generon, UK) according to the manufacturer's instruction before administration of the nanobodies to animals.

**Binding experiments by TR-FRET**. HEK293 cells (ATCC CRL-1573) were transfected with rat SNAP-tagged mGlu2 receptors (Maurel et al. Nature Methods 2008). 24 h after transfection, cells were labeled in 150 mm cell culture plate with 100 nM SNAP-Lumi4Tb in DMEM-GlutaMAX for 1 h at 37°C, and then washed 3 times with Krebs buffer (10 mM Hepes pH7.4, 146 mM NaCl, 4.2 mM KCl, 1 mM CaCl2, 0.5 mM MgCl2, 5.6 mM glucose and bovine serum albumin 0.1%). Cells were detached using cell dissociation buffer (Sigma aldrich) transferred at 10 000 cells per well in a white 384-well plate (Greiner bio-one, PS, F-bottom, small volume, Hibase, medium binding). Increasing concentration of His-tagged nanobodies together with either 1 µM LY379268 (Tocris, Bio-techne, France) or 1 µM LY341495 (Tocris, Bio-techne, France) and with 200 nM of anti-6His-d2 (Tocris, Bio-techne, France) were applied on Lumi4Tb-labeled cells in a final volume of 20 µL and incubated overnight at 20°C. FRET signal was determined by measuring the sensitized acceptor emission (665 nm) and Tb donor emission (620 nm) using a 50 µs delay and 450 µs integration time upon excitation at 337 nm on a PHERAstar FS plate-reader (BMG LabTech, Ortenberg, Germany). TR-FRET (or HTRF) ratio (665 nm/620 nm × 10⁴, Cisbio patent 8 US5,527,684) was calculated for preventing interferences due to medium variability and chemical compound or to normalize experiments when using cells expressing different receptor levels.

**Measurements of inositol phosphate**. HEK293 cells were co-transfected with rat SNAP-tagged mGlu2 receptors, GqTop and EAAC1 by electroporation and transferred at 100 000 cells per well in a black 96-well plate (Greiner bio-one, PS, F-bottom). 24 h after transfection at 37° C, cells were washed and stimulated with 0.6 nM of agonist LY379268 (EC20) and various concentration of nanobodies and then incubated for 30 min at 37°C, 5% CO2. The measurement of inositol phosphate-1 accumulation in HEK293 was determined using the IP-One HTRF kit (Cisbio) according to the manufacturer's recommendation.

**Fluorescent nanobody labeling**. Nanobodies were dialyzed overnight at 4°C in phosphate buffer (50 mM pH 8) and incubated (250 µg of nanobodies at 2 mg.mL⁻¹) with the fluorophore NHS (d2-NHS (Cisbio, Codolet, France)) into phosphate buffer (100 mM pH 7) at a ratio of 6 (fluorophore / VHH), for 45 min at 25°C. Nanobodies were then purified by gel filtration column (NAP-5, GE Helthcare, Little Chalfont, UK) in phosphate buffer 100 mM pH 7. The final molar ratio of fluorophores per nanobodies was calculated as the fluorophore concentration / conjugated nanobody concentration, and the conditions were set up for a ratio between 2 and 3. The concentration of fluorophores in the labeled fraction was calculated as the OD/ε (OD at 650 nm and ε = 225,000 M⁻¹cm⁻¹ for d2), while that of nanobodies was determined by the OD280. The conjugated concentration calculated as OD280 - (OD650/Rzmax)/ε nanobody with Rzmax = OD650/OD280. Purified labeled fraction were supplemented with 0.1% BSA and kept at -20°C.

**Histological processing of brains**. Wild-type C57BL/6J mice were purchased from Janvier Labs and brains from mGlu2-/- mice were kindly provided by Dr. Gonzalez-Maeso. Mice were anesthetized (Euthasol Vet 8%, Alcyon, Pau, France) and sacrificed by a cardiac perfusion of 4% paraformaldehyde (PFA). Brains were extracted and fixed in a 4% PFA solution (Euromedex, Souffelweyersheim, France) overnight at 4° C. Brains were rinsed in PBS and cryoprotected in a 30% sucrose solution for 4 days at 4°C. Brains were then included in OCT (Tissue-Tek, Sakura Finetek, Villeneuve d'Asq, France), quickly frozen in acetone chilled on dry ice and conserved at -80°C until use. Frozen brains included in OCT, were put at -20°C 24 h prior cryo-sectioning. They were then mounted on a cryostat (Leica, Nanterre, France) and 16 µm coronal or sagittal sections were performed and directly mounted on Superfrost Plus glass slides (Microm) and kept at -20°C, until use. For immunohistofluorescence experiments, coronal and sagittal brain tissue sections of WT and mGlu2^{-/-} mice were blocked 1 h at 25°C with a blocking solution (3% BSA, 0.1% Triton X100 in PBS) and incubated with IGF1202-d2 (200 nM), overnight at 4°C, to label mGlu2 receptors. Sections were washed with PBS with distilled water and mounted using Fluoroshield containing DAPI to stain nuclei (Sigma-Aldrich). Images were taken using a slide scanner axio scan Z1 microscope (Zeiss, Jena, Germany) by performing full-section mosaic with a 20x enlargement. For immunohistochemistry experiments, C57BL/6J female WT mice were injected i.p. either with 10 mg/kg of IGF1202 or with an equivalent volume of PBS (150 µL) as a negative control. 24 h post-injection, mice were anesthetized (Euthasol Vet 8%) and sacrificed as described above. Ten glass slides per animal, each containing 5 brain tissue sections, with 80 µm spacemen between each section, were rinsed with TBS and incubated 1 h at 25°C with a blocking solution (5% normal goat serum - 0.1% Triton X100 - TBS). Sections were then incubated with a monoclonal rabbit anti-6His (1:500, Life Technologies) 2 h at 25°C, washed in TBS. After blocking endogenous peroxidase (3% H₂O₂ solution, 20 min at 25°C) sections were incubated with a goat anti-rabbit biotinylated antibody (1:100, ABC peroxidase kit, Vector Laboratories). Labeling was revealed by using 3-3'-diaminobenzidine chromogen solution (DAB) according to the instructions provided in the DAB peroxidase substrate kit (Vector Laboratories). Sections were then washed in distilled water and mounted using Fluoroshield with DAPI. Images were taken using a slide scanner axio scan Z1 microscope by performing full-section mosaics with a 20x enlargement.

Nanobody quantification in blood. A quantitative test of nanobody concentration has been developed based on the HTRF-binding assay on cells expressing Snap-tagged mGlu2 receptor. To determine the linearity of the assay, increasing concentrations of IGF1168 or IGF1202 were added to 10.000 cells expressing Lumi4Tb labeled SNAP-tagged mGlu2, 200 nM of anti-6His-d2 antibody and 1 µM LY379268 in a 384 well plate. After one night incubation at 20°C, the HTRF signal was recorded on a Pherastar FS plate reader and the linearity determined using Graphpad Prism. C57BL/6J WT mice (Janvier Lab) were treated by i.p. administration of 10 mg/kg of IGF1168, IGF1202 or an equivalent volume of PBS (150 µL) and blood samples were collected at different time point (30 min, 1 h, 2 h, 4 h, 6 h, 24 h and 48 h). The plasma was extracted using microvette (microvette CB 300 K2E, Sarstedt, Marnay, France) and frozen until use. Plasma (3 µL) was applied to cells expressing mGlu2 receptor, labeled with SNAP-Lumi4Tb in the presence of 200 nM of anti-6His-d2. FRET signal was determined as indicated for saturation binding assay.

**Nanobody radiolabeling.** IGF1202 was radiolabeled using bacterial transglutaminase and a synthetic peptide tag, CBz-Gln-Gly-Lys-OH. CBz-Gln-Gly-Lys-OH was synthetized using manual solid phase peptide synthesis on a microwave synthesizer (CEM corporation) following a standard Fmoc-based synthesis protocol. Starting from the Fmoc-Lys(Boc)-Wang resin loaded at 0.32 mmol/g (Novabiochem) the synthesis was performed on a 0.015 mmol/reactor scale. Protected amino acids Fmoc-Gly-OH and CBz-Gln-OH (5 equiv.) were coupled using HATU (4.5 equiv.) and iPr2EtN (10 equiv.) in DMF for 20 minutes at 60°C. Fmoc group was deprotected by three successive treatments with 20% piperidine in DMF for 3 minutes. The peptide was then deprotected and cleaved from the resin by a treatment with trifluoroacetic acid/H2O/triisopropylsilane (95/2.5/2.5) for 2 h at 25°C. The peptide was then precipitated by dilution into an ice-cold diethyl ether solution, recovered by centrifugation and washed twice with diethyl ether. CBz-Gln-Gly-Lys-OH was tritiated using the following procedure. To a solution of the CBz-protected peptide (87 nmol, 1 equiv.) in DMF (50 µL) at 25°C was added iPr2EtN (870 nmol, 10 equiv.) and [³H]-N-(propionyloxy)succinimide (87 nmol, 1 equiv.). The mixture was stirred at 25°C for 2 h. The labeled product was purified by HPLC equipped with a Luna Omega C18 (150 × 4.6 mm; 3 µm) column (Phenomenex) with a gradient of 0-100% buffer B in buffer A over 20 minutes with a flow rate of 1 mL/min (buffer A: 0.1% formic acid in H2O, buffer B: 0.1% formic acid in acetonitrile). The radiolabeling of IGF1202 through lysine conjugation was carried out in PBS buffer at pH=7.4 using 1U/20 nmol of bacterial transglutaminase (Zedira), 2 equivalents of the radiolabeled peptide tag and at a concentration of 80 µM of IGF1202 protein. The mixture was then incubated for 2 h at 25°C. After incubation, the labeled protein was purified on Protino Ni-NTA resin (Macherey-Nagel) and then buffer was exchanged to NaCl 0.9% using Amicon centrifugal filter (Merck) for in vivo experiments. Part of the labeled IGF1202 was digested by trypsin to identify Lys121 as the conjugation site by MALDI-TOF (Applied Biosystems) analysis.

***In vivo* cerebral quantification and localization of the tritiated nanobody**. Experiments were conducted on adult female WT C57BL/6J mice (21.5 ± 0.3 g) purchased from Janvier Labs. Mice were injected intraperitoneally with 10 mg/kg of [³H]-IGF1202 in 50 µL. Animals were euthanized 4 h, 24 h, 48 h, 4 days or 7 days after administration, perfused with NaCl 0.9% (10 mL/mouse, 200 mL/h) for brain collection and freezing. Brains were used for either tissue quantification, or cerebral localization of β-radioactivity. Quantification of β-radioactivity contained in total brain homogenate was performed using a Liquid Scintillation Analyzer (Tri-Carb 2100TR, Packard BioScience Company, USA). Brain tissue sections (20 µm thickness) were prepared on a microtome to perform imaging and observe cerebral localization by digital autoradiography with a β-IMAGER (Biospace Lab, France).

**Novel object recognition test on a neurodevelopmental mouse model of schizophrenia induced by PCP administration**. The mice used were C57BL/6J females and males, from the Janvier Labs. These mice were injected, subcutaneously, with 10 mg/kg of phencyclidine (PCP mice) (Sigma Aldrich, ANSM authorization #A-2018-3-330-S) or with a saline solution (NaCl 0.9%) (vehicle mice) at P7, P9 and P11. One week before the test, mice were extensively handled by the operator: the first two days, the operator put his hand on the home cage of the animals to familiarize the animals to his presence and the following days, the animals were handled few minutes per day. For novel objects recognition (NOR), the testing was carried out in an open field, consisting of 4 compartments of 50 * 50cm, each containing 1 mouse, placed in a dimly lit room with clearly visible contextual cues (black on white patterns) on the surrounding walls. Each mouse was habituated to the arena for 10 min the day before the training session. Mice then performed the novel object recognition task. After a 10-min training session, a 24 h retention interval was observed, during which the mice were transferred back to the home cage and the arena and objects were cleaned. During the test session, the animals were placed back into the arena in presence of an identical copy of one of the familiar objects and a novel object and allowed to explore the objects freely for 10 min. The objects were plastic toys (approximately 3-cm width, 3-cm length, 5-cm height) and were cleaned with 10% ethanol between sessions. The experiments were video-recorded and exploration times (nose in contact or sniffing at < 1 cm) were measured by a blinded observer. Mice with total time exploration of less than 3 sec in the test session were excluded. Discrimination indexes (exploration time of novel object - exploration time of familiar object) / total object exploration time) were compared between groups. Drug administration: i.p. administration of LY379268 (1 mg/kg) was done 3 h before the test. i.p. administration of IGF1202 (10 mg/kg) was done , 3 h before the training session with the objects. i.c.v administration of IGF1202 or IGF1159 (4 pmol in 5 µL) was done 24 h before the training session through a cannula that was implanted 7 days earlier. Cannulas implantation was performed on mice anesthetized with a mixture of ketamine (Imalgene 500, 50 mg/ml, Merial), and xylazine (Rompun 2%, 20 mg/ml, Bayer) diluted in 0.9% (w/v) NaCl solution (saline) (2:2:1, i.p., 0.1 ml/30 g) and mounted on a stereotaxic apparatus using flat skull coordinates. Stainless steel guide cannula (22 gauge, 5.00 mm, World Precision instruments, US) were implanted in the ventricle (A/P = -0.5 mm; M/L = 1.1 mm; D/V = -2.2 mm). Cannulas were implanted vertically in the coronal plane to avoid damage to the wall of the lateral ventricle. The guide cannula was fixed to the skull with anchor screws and dental acrylic (AgnTho's). Mice could recover for a minimum of 7 days prior to behavioral testing.

**Statistical analyses**. All quantitative data were initially analyzed using Shapiro-Wilk's normality test using GraphPad Prism 8 (GraphPad software Inc, San Diego, CA, USA). When data sets were normally distributed parametric tests were performed: the one-way ANOVA or the two-way ANOVA. When data sets were not normally distributed (p < 0.05) nonparametric tests were used: Krustal-Wallis (Dunn's *post-hoc* analysis). A probability of 0.05 has been defined as a significant difference for all statistical analysis.

### Example 2: The optimized bivalent biparatopic IGF1202 nanobody

To increase the likehood to observe a pharmacological effect in the brain even at a low nanobody concentration, our rational was to improve the affinity and the potency in enhancing the mGlu2 receptor activation of IGF1168. We generated and screened a number of bivalent nanobodies using IGF1168, IGF1159 and IGF1166 and two linkers, and tested them for their affinity and potency on mGlu2 (**Figure 4**). It is a strategy known to increase the avidity or the residence time of the nanobody on its target or both. We selected the bivalent biparatopic IGF1202 that showed a 10-fold increase in the apparent affinity for mGlu2 compared to IGF1168 (0.30 ± 0.04 nM and 4.93 ± 0.60 nM, respectively) in the presence of agonist (LY379268 1 µM) (**Figure 1a****,** **4a**). In addition, we measured almost a two-logs increase for IGF1202 (pEC50 = 9.16 ± 0.05) compared to IGF1168 (pEC₅₀ = 7.22 ± 0.06) in their PAM potency of enhancing mGlu2 activation by a low agonist concentration (**Figure 1b****,** **4b**).

To validate the specificity of IGF1202 in the brain, we performed *ex vivo* immunohistofluorescence using a red-labeled IGF1202 that preserved the binding properties. Sagittal brain tissue sections from wild-type C57BL/6J mice revealed mGlu2 labeling in regions known to highly express this receptor including the prefrontal cortex, the hippocampus, the striatum, the cerebellum and the olfactory bulb. No labeling was detected in brain tissue sections from mGlu2^{-/-} mouse (**Figure 1c**), demonstrating the specificity of IGF1202 for the mGlu2 receptor in the brain.

### Example 3: The nanobody IGF1202 penetrates the brain

For *in vivo* experiments, we chose the i.p. route to administer the nanobody in a similar fashion to small molecules. We first evaluated the lifetime of IGF1202 and IGF1168 in the blood stream, since they are likely eliminated quickly by the kidney due to their small size. We collected blood samples after i.p. administration of 10 mg/kg of nanobodies and quantified the amount of nanobody still present at different time points by developing a time-resolved FRET-based assay (**Figure 5**). IGF1202 was detected for 12 h after administration, longer than the smaller size IGF1168 that was totally excluded from the blood after 6 h.

We questioned whether a fraction of the administered IGF1202 could reach the brain, as reported for some nanobodies (Li et al., 2012). We used the high sensitivity of the radioactivity and IGF1202 was tritiated on a single lysine using bacterial transaminase and CBz-Gln-Gly-Lys([³H]Pr)-OH peptide, a derivatization strategy that preserved the binding properties on mGlu2 receptor. We monitored over 7 days the presence of [³H]-IGF1202 in brains collected from mice at several time points after a 10 mg/kg i.p. administration. [³H]-IGF1202 was detected in brain homogenates at all time points reaching a maximum at 4 h post administration with 0.08% of the administered radioactivity detected in the total brain and still significantly present 7 days after administration (**Figure 2a**). Considering a homogenous nanobody distribution in the whole brain volume (about 500 mm³), IGF1202 brain concentration reaches 4 h post administration 9.2 nM, which is over 30 times the Kd, and getting down to 1.5 nM 7 days after administration that is still over 5 times the Kd. The local concentration should even be higher near mGlu2 expressing neurons as they would trap the circulating nanobody limiting the diffusion and also considering that IGF1202 remains in the extracellular space. To gain more qualitative analysis of IGF1202 brain penetration and distribution, we used immunohistochemistry. 24 h after 10 mg/kg of IGF1202 administration through the i.p. route, mice were sacrificed, brains were perfused, fixed and brain tissue sections were prepared. After labeling using an anti-6his antibody and a secondary antibody coupled to the horseradish peroxydase (HRP), IGF1202 is detected in the brain notably in the ventral tegmental area (VTA) and in the cortex (**Figure 2b****,** **Figure 6b**). As a negative control, no immunoreactivity was detected in the brain of mice injected with an equivalent volume of PBS (**Figure 2b****,** **Figure 6a**). Altogether, these observations indicate that IGF1202 penetrates the brain where it is detected in mGlu2 expressing areas and remains present for several days.

### Example 4: IGF1202 improves the recognition memory in PCP-treated mice

To test the efficacy of IGF1202 *in vivo,* we used a neonatal phencyclidine (PCP) mouse model that causes impairments in learning and recognition memory (Nakatani-Pawlak et al., 2009). In this model, administration of the NMDA receptor antagonist PCP, at postnatal day 7, 9 and 11, induces cognitive deficits that can be measured with the novel object recognition (NOR) test, which is indicave of the animal ability to discriminate a novel object from a familiar one (**Figure 3**). First, we evaluated the effect of IGF1202 (4 pmol in 5 µL) in restoring cognition when injected into the brain through cannulas implanted into the third ventricle. It enables to precisely control the quantity of nanobodies delivered into the brain. As expected, PCP-treated mice were less performant in the NOR test compared to vehicle controls (**Figure 3**). IGF1202 restored the cognitive capacity of these mice as efficiently as the mGlu2/mGlu3 orthosteric agonist LY379268 (1 mg/kg). Interestingly, IGF1202 was injected the day before the habituation and learning phases, i.e. 48 h before the recognition test, while LY379268 was injected just 1 hour before the test. As a control, we used IGF1159, a nanobody that binds to mGlu2 but does not modulate its signaling. Administration of IGF1159 into the brain (4 pmol in 5 µL) failed to ameliorate the NOR score of PCP-treated mice. In addition, we determined that neither IGF1202 nor IGF1159 or LY379268 altered the memory in control mice (**Figure** 7). Altogether these data indicate that a single administration of IGF1202 into the brain 2 days prior the experiment is as efficient as an acute administration of the orthosteric mGlu2/mGlu3 agonist to ameliorate cognitive function in this model.

Because IGF1202 penetrates the brain when injected i.p., we next analyzed whether peripheral injection of the bivalent nanobody could also restore the cognitive capacity of PCP-treated mice. i.p. injection of IGF1202 (10 mg/kg) was sufficient to increase the score of PCP-treated mice in the NOR task with an effect similar to LY379268 or to i.c.v. administered IGF1202 (**Figure 3**). The i.p. injection was done prior the learning session and 24 h and also at 7 days before the test. This supports that IGF1202 modulates mGlu2 receptor activity after brain penetration at least 24 h after peripheral administration.

### Discussion

Using imaging, biophysical and behavioral experiments we showed that the nanobody IGF1202 penetrates the brain during the first hours after i.p. injection and has a long-lasting residency in the brain. Low brain penetration is raised as an obstacle for the use of immunoglobulins to treat human brain diseases. Their estimated brain uptake is roughly 0.1-0.2% of the peripherally administrated dose, eventually reaching a higher rate in physio-pathological conditions that induce BBB damage. The rate of IGF1202 brain penetration is in a similar range, reaching 0.08% at 4 h and remains stable for a few days. However, considering the quantity of compound administered to mice (10 mg/kg for IGF1202, 3 to 25 mg/kg for IgG) and their size difference (30 kDa and 150 kDa, respectively), a larger number of nanobody molecules are present in the brain. In addition, thanks to their small size, nanobodies have better tissue penetration than whole antibodies, which implicates easier access to its target. In addition, their small size confer them access to small cavities revealing additional epitopes than immunoglobulines. Nanobodies are also interesting in the way that they show no or very low immunogenicity, are very stable and are easily produced.

mGlu2 receptor is a validated target for the treatment of symptoms associated to schizophrenia. Despite the failure of the mGlu2 compounds in phase II or phase III by lack of efficiency (Patil et al, 2007; Adams et al, 2013; Salih et al (2015) ; Litman et al (2016)), a recent *post-hoc* analysis indicates that an epigenetic down-regulation of mGlu2 receptor expression occurs after anti-psychotic treatment. This has likely masked the effect of the compounds in some patients while other patients showed positive effects, giving new hopes for mGlu2 targeting compounds in schizophrenia. Based on our present results, IGF1202 represents an interesting alternative to small molecules. Compared to small molecule PAMs, the nanobody has the advantage to be hydrophilic limiting eventual side effects due to off-target binding reported for those highly hydrophobic PAMs. Compared to orthosteric agonists, IGF1202 has the advantages to be specific for mGlu2 receptor and to potentiate the activation only where and when the endogenous ligand is present avoiding over-stimulation and desensitization often associated to side effects.

Altogether, these results open new avenues for the handling of brain diseases with nanobody-based immunotherapies with peripheral and low frequency administration of the treatment. In addition, IGF1202 will help to apprehend cognitive deficits associated to schizophrenia and to ascertain the relevance of mGlu2 receptor activation to treat patients.

### REFERENCES

Adams, D. H. et al. A long-term, phase 2, multicenter, randomized, open-label, comparative safety study of pomaglumetad methionil (LY2140023 monohydrate) versus atypical antipsychotic standard of care in patients with schizophrenia. BMC Psychiatry 13, 143 (2013).
Doumazane, E. et al. A new approach to analyze cell surface protein complexes reveals specific heterodimeric metabotropic glutamate receptors. FASEB J 25, 66-77 (2011).
Gerbier R, Alvear-Perez R, Margathe JF, Flahault A, Couvineau P, Gao J, De Mota N, Dabire H, Li B, Ceraudo E, Hus-Citharel A, Esteoulle L, Bisoo C, Hibert M, Berdeaux A, Iturrioz X, Bonnet D, Llorens-Cortes C. Development of original metabolically stable apelin-17 analogs with diuretic and cardiovascular effects. FASEB J 31 (2):687-700 (2017).
Hutt M, Farber-Schwarz A, Unverdorben F, Richter F, Kontermann RE. Plasma half-life extension of small recombinant antibodies by fusion to immunoglobulin-binding domains. J Biol Chem 287 (3), 4462-4469 (2012).
Karlin S, Altschul SF. Methods for assessing the statistical significance of molecular sequence features by using general scoring schemes. Proc Natl Acad Sci U S A 87(6):2264-8 (1990).
Li, T. et al. Cell-penetrating anti-GFAP VHH and corresponding fluorescent fusion protein VHH-GFP spontaneously cross the blood-brain barrier and specifically recognize astrocytes: application to brain imaging. FASEB J 26, 3969-3979 (2012).
Litman, R. E. et al. AZD8529, a positive allosteric modulator at the mGluR2 receptor, does not improve symptoms in schizophrenia: A proof of principle study. Schizophr. Res. 172, 152-157 (2016).
Maurel et al., 2008. Cell-surface protein-protein interaction analysis with time-resolved FRET and snap-tag technologies: application to GPCR oligomerization. Nature Methods volume 5, pages561-567 (2008).
Nakatani-Pawlak, A., Yamaguchi, K., Tatsumi, Y., Mizoguchi, H. & Yoneda, Y. Neonatal phencyclidine treatment in mice induces behavioral, histological and neurochemical abnormalities in adulthood. Biol. Pharm. Bull. 32, 1576-1583 (2009).
Patil, S. T. et al. Activation of mGlu2/3 receptors as a new approach to treat schizophrenia: a randomized Phase 2 clinical trial. Nat Med 13, 1102-1107 (2007).
Salih, H. et al. Pharmacokinetic and pharmacodynamic characterisation of JNJ-40411813, a positive allosteric modulator of mGluR2, in two randomised, double-blind phase-I studies. J. Psychopharmacol 29, 414-425 (2015).
Scholler, P. et al. Allosteric nanobodies uncover a role of hippocampal mGlu2 receptor homodimers in contextual fear consolidation. Nat Commun 8, 1967 (2017).
Stork R, Campigna E, Robert B, Müller D, Kontermann RE. Biodistribution of a bispecific single-chain diabody and its half-life extended derivatives. J Biol Chem 18;284(38):25612-9 (2009).
Walker A, Dunlevy G, Rycroft D, Topley P, Holt LJ, Herbert T, Davies M, Cook F, Holmes S, Jespers L, Herring C. Anti-serum albumin domain antibodies in the development of highly potent, efficacious and long-acting interferon. Protein Eng Des Sel 23 (4):271-8 (2010).

## Claims

1. A biparatopic nanobody comprising
(i) one single domain antibody having
a CDR1 having a sequence set forth as SEQ ID NO:1, a CDR2 having a sequence set forth as SEQ ID NO:2and a CDR3 having a sequence set forth as SEQ ID NO:3; or
a CDR1 having a sequence set forth as SEQ ID NO:4, a CDR2 having a sequence set forth as SEQ ID NO:5 and a CDR3 having a sequence set forth as SEQ ID NO:6; or
a CDR1 having a sequence set forth as SEQ ID NO:7, a CDR2 having a sequence set forth as SEQ ID NO:8 and a CDR3 having a sequence set forth as SEQ ID NO:9; preferably
a CDR1 having a sequence set forth as SEQ ID NO:1, a CDR2 having a sequence set forth as SEQ ID NO:2 and a CDR3 having a sequence set forth as SEQ ID NO:3; and
(ii) another single domain antibody having
a CDR1 having a sequence set forth as SEQ ID NO:10, a CDR2 having a sequence set forth as SEQ ID NO:11 and a CDR3 having a sequence set forth as SEQ ID NO:12; or a CDR1 having a sequence set forth as SEQ ID NO:13, a CDR2 having a sequence set forth as SEQ ID NO:14 and a CDR3 having a sequence set forth as SEQ ID NO:15; or a CDR1 having a sequence set forth as SEQ ID NO:16, a CDR2 having a sequence set forth as SEQ ID NO:17 and a CDR3 having a sequence set forth as SEQ ID NO:18.

2. The biparatopic polypeptide of claim 1 comprising (i) one single domain antibody having a CDR1 having a sequence set forth as SEQ ID NO:1, a CDR2 having a sequence set forth as SEQ ID NO:2 and a CDR3 having a sequence set forth as SEQ ID NO:3; and (ii) another single domain antibody having a CDR1 having a sequence set forth as SEQ ID NO:16, a CDR2 having a sequence set forth as SEQ ID NO:17 and a CDR3 having a sequence set forth as SEQ ID NO:18.

3. The biparatopic polypeptide of claim 2, comprising one single domain antibody having at least 85% identity, preferably at least 90% identity, more preferably at least 95% identity, and even 100% identity with the sequence set forth as SEQ ID NO:19 , and another single domain antibody having at least 85% identity, preferably at least 90% identity, more preferably at least 95% identity, and even 100% identity with the sequence set forth as SEQ ID NO: 24.

4. The biparatopic nanobody according to anyone of claims 1 to 3, wherein the both single domains are linked together with a linker sequence having at least 80% of identity with the sequence set forth as SEQ ID NO:25, preferably 100% identity with SEQ ID NO: 25 (EPKIPQPQPKPQPQPQPQPQPKPQPKPEP), or any other sequence selected from sequences set forth as SEQ ID NO:26 to SEQ ID NO: 31.

5. The biparatopic nanobody according to anyone of claims 1 to 4, comprising an amino-acid sequence having at least 80% of identity with the sequence set forth as SEQ ID NO:32 or 33.

6. The biparatopic nanobody according to anyone of claims 1 to 5 comprising a further domain, such as a peptide, a nanobody or a fluorinated chain, and in particular a further domain directed against a serum protein, preferably a serum albumin.

7. A nucleic acid encoding for a biparatopic nanobody according to claim 5, preferably a nucleic acid having at least 80% of identity with the sequence set forth as SEQ ID NO:40 or 41.

8. A vector which comprises the nucleic acid of claim 7.

9. A host cell which is transformed with the nucleic acid sequence of claim 6 or with the vector of claim 8.

10. The biparatopic nanobody according to anyone of claims 1 to 6 for use as a medicament.

11. A pharmaceutical composition comprising a biparatopic nanobody according to anyone of claims 1 to 6, and a pharmaceutical excipient.

12. The pharmaceutical composition of claim 11, wherein it comprises another mGluR2 agonist or another agent for the treatment of a neurological or psychiatric disorder.

13. The biparatopic nanobody according to anyone of claims 1 to 6 or the pharmaceutical composition of claim 11 or claim 12, for use in a method of treating a patient suffering from a neurological or psychiatric disorder associated with glutamate dysfunction.

14. The biparatopic nanobody according to anyone of claims 1 to 6 or the pharmaceutical composition of claim 11 or claim 12 for use in a treatment according to claim 13, wherein the neurological or psychiatric disorder associated with glutamate dysfunction is selected from the group consisting of cerebral ischemia, head trauma, neurodegeneration, Alzheimer's disease, epilepsy, and pain, or or psychiatric disorder associated with glutamate dysfunction is selected from the group consisting of psychosis, schizophrenia, anxiety, depression, and substance-related disorder or addiction/drug or alcohol dependence.

15. The biparatopic nanobody according to anyone of claims 1 to 6 which is conjugated with a detectable label.

16. The biparatopic nanobody of claim 15 wherein the label is selected from the group consisting of radioisotope labels, fluorescent labels, chemiluminescent labels, enzyme labels, and bio luminescent labels.

17. An *in vitro* method of detecting the activation of mGluR2 in a sample comprising the steps of i) contacting the sample with a biparatopic nanobody conjugated with a detectable label as defined in claim 15 or 16, ii) and detecting the binding of said biparatopic antiboby to said sample wherein said detection is indicative of the activation of mGluR2.
